# EUROPEAN PATENT APPLICATION

(11) **EP 1 777 222 A1**
(43) Date of publication of application: **25.04.2007**
(21) Application number: 05020721.6
(22) Date of filing: 22.09.2005
(51) Int. Cl.: C07D 307/91, C07D 491/06, A61K 31/343, A61K 31/55, A61P 25/28, C07D 307/00, C07D 223/00

(54) **Cholinergic enhancers with improved blood-brain barrier permeability for the treatment of diseases accompanied by cognitive impairment**

(71) Applicant: Galantos Pharma GmbH, 55131 Mainz (DE)
(72) Inventor: Maelicke, Alfred, Prof. Dr., 55268 Nieder-Olm (DE); Weichel, Carolin, 20259 Hamburg (DE)
(74) Representative: polypatent

(57) **Abstract**

The present invention refers to compounds that, in addition to enhancing the sensitivity to acetylcholine and choline of neuronal cholinergic receptors and/or acting as chvlinesterase inhibitors and/or neuroprotective agents, have enhanced blood-brain barrier permeability in comparison to their parent compounds. The compounds are derived (either formally by their chemical structure or directly by chemical synthesis) from natural compounds belonging to the class of amaryllidaceae alkaloids e.g. galanthamine, narwedine and lyeoramine, or from metabolites of said compounds. The compounds of the present invention can either interact as such with their target molecules, or they can act as "prodrugs", in the sense that after reaching their target regions in the body they are converted by hydrolysis or enzymatic attack to the original parent compound and react as such with their target molecules, or both. The compounds of this invention may be used as medicaments for the treatment of human brain diseases associated with a cholinergic deficit, including the neurodegenerative diseases Alzheimer's and Parkinson's disease and the psychiatric diseases vascular dementia, schizophrenia and epilepsy.

## Description

The present invention refers to compounds that, in addition to enhancing the sensitivity to acetylcholine and choline, of neuronal cholinergic receptors, and/or acting as cholinesterase inhibitors and/or neuroprotective agents, have enhanced blood-brain barrier permeability in comparison to their parent compounds. The compounds are derived (either formally by their chemical structure or directly by chemical synthesis) from natural compounds belonging to the class of amaryllidaceae alkaloids e.g. Galanthamine, Narwedine and Lycoramine, or from metabolites of said compounds. The compounds of the present invention can either interact as such with their target molecules, or they can act as "pro-drugs", in the sense that after reaching their target regions in the body, they are converted by hydrolysis or enzymatic attack to the original parent compound and react as such with their target molecules, or both. The compounds of this invention may be used as medicaments for the treatment of human brain diseases associated with a cholinergic deficit, including the neurodegenerative diseases Alzheimer's and Parkinson's disease and the neurological/psychiatric diseases vascular dementia, schizophrenia and epilepsy.

The diffusion of compounds from the blood plasma into the brain is complicated by the presence of the blood-brain barrier that is a membrane that segregates the brain interstitial fluid from the circulating blood. In designing drugs active in the central nervous system and able to cross the blood-brain barrier, one can exploit endogenous active mechanisms, utilize proper delivery techniques or modify the chemical structure through the synthesis of pro-drug derivatives.

Galanthamine is an alkaloid that can be isolated from the bulbs of various snowdrop (Galanthus) and narcissus species (daffodils, Amaryllidaceae). Synthetic Galanthamine hydrobromide is manufactured by, among other companies, Sanochemia and Janssen Pharmaceutics. The drug has been approved in more than 70 nations for the treatment of mild-to-moderate Alzheimer's disease (AD), a neurodegenerative brain disease. Extensive studies of the pharmacokinetic profile, tissue distribution and accumulation of Galanthamine in mice, rats, rabbits and dogs have shown that Galanthamine given orally is by no means preferentially distributed to the brain where it is supposed to exert its therapeutic activity in said brain diseases. In contrast, it is accumulated at much higher concentrations in other body tissues. In male and female rat tissues the highest concentrations are observed in kidney (tissue to plasma ratio; T/P ~10-15), salivary and adrenal gland (T/P ~ 7-14), female rat spleen (T/P ~ 20), lung, liver, heart, skeletal muscle and testes (T/P ~ 2-4). In contrast, the brain to plasma ratio is only T/P ~ 1.5. Similarly, the brain/plasma partition coefficient K_{brain} is significantly lower than most other K_{organ} of Galanthamine.

Limited penetration ability of Galanthamine through the blood-brain barrier (BBB) into the central nervous system (CNS) is indicated also by the compound's logP value of 1.3, logP being defined as the decadic logarithm of the partition coefficient P which is the ratio of the concentration of compound in aqueous phase to the concentration of compound in immiscible solvent, as the neutral molecule. The logP value is obtained by predictive computational methods and provides a general guideline as to whether a drug gains rapid access to the CNS, or not. Thus, it has been established over the past more than 30 years that, assuming passive absorption, drugs with optimum CNS penetration generally have logP values around or somewhat above 2. Significantly lower logP values are often associated with low brain-to-plasma and high non-brain tissue-to-plasma ratios (see above: logP and T/P ratios for Galanthamine). However, much higher logP values are also of disadvantage, as high lipophilicity is often associated with toxicity, non-specific binding, insufficient oral absorption and limited bioavailability. It follows from this account that BBB penetration and T/P ratios are essential parameters to be considered in the case of drugs that are supposed to act mainly or exclusively in the central nervous system.

Other important parameters controlling BBB penetration of a compound are the total polar surface area, the existence of ionisable groups on the molecule and the affinity of binding to biological membranes as compared to the affinity of binding to serum albumin. The latter data set is often used to scrutinise calculated logP values. In those cases in which special transport systems do not play a major role for the transport of a compound through the BBB, the predictions of lipophilicity and BBB penetration properties are quite suitable for the design of derivatives that transfer the BBB more efficiently than the parent compound.

The present invention refers to methods by which the lipophilicity and/or BBB penetration and/or brain-to-plasma ratio of a compound is enhanced by formation of a reversible linkage with one or more suitable groups so as to yield "pro-drugs", i.e. chemical derivatives that, after having passed through the blood-brain barrier, are converted (back) to the original compound itself inside the patients brain. Liberation of the parent compound may be by chemical hydrolysis or enzymatic attack. In another embodiment, the present invention refers to compounds that after chemical modification of the base compound have achieved a lopP value more favourable for BBB penetration, with these derivatives acting as such at their target molecules in the patient's brain.

Presently approved drugs for the treatment of Alzheimer's disease (AD) have in common that they all target excitatory neurotransmission in the brain, namely the cholinergic and the glutamatergic systems. Three of the four presently available drugs (Donepezil, Rivastigmin, Galanthamine, Memantine) are cholinergic enhancers (Donepezil, Rivastigmin, Galanthamine) in that they all inhibit the family of acetylcholine-degrading enzymes denoted as cholinesterases (ChE). Inhibition of ChE increases the synaptic concentrations of acetylcholine (ACh), thereby enhancing and prolonging the action of ACh on muscarinic (mAChR) and nicotinic (nAChR) acetylcholine receptors. In addition to acting as ChE inhibitor, Galanthamine also acts by allosterically stimulating (sensitising) cholinergic receptors. Allosteric sensitisation of nicotinic receptors enhances their activation by ACh or choline (Ch), thereby correcting for a disease-associated deficit in transmitter or receptor concentration (Maelicke A & Albuquerque EX (1996) Drug Discovery Today 1, 53-59; Maelicke A & Albuquerque EX (2000) Eur J Pharmacol 393, 165-170). In addition to their therapeutic benefits, these drugs induce adverse peripheral and central side effects; the muscarinic ones including nausea, vomiting and diarrhea, and the nicotinic ones including tremors and muscle cramps. From meta data (Cochrane reviews, (2004), Issue 4) and direct comparison clinical studies (Wilcock GK et al. (2000) Brit Med Journ 321:1-7), the relatively weakest of the three presently used ChE inhibitors, Galanthamine, has the highest clinical efficacy, with the therapeutic benefit achieved at concentrations that are well below those required for effective inhibition of AChE (Raskind MA et al. (2000) Neurology 54, 2261-2268 ; Maelicke A & Albuquerque EX (2000) Eur J Pharmacol 393, 165-170). It has been suggested that the stronger therapeutic efficacy of Galanthamine, as compared to the other two available ChE inhibitors, is due to an additional or alternative mode of action, i.e. allosteric sensitisation of nAChR (Maelicke A & Albuquerque EX (1996) Drug Discovery Today 1, 53-59).

Galanthamine enhances nicotinic cholinergic neurotransmission by acting directly on nicotinic receptors (Schrattenholz A et al. (1996) Mol Pharmacol 49, 1-6; Samochocki M et al. (2003) J Pharmacol Exp Therap 305, 1024-1036). The drug binds to a distinct allosteric site on these receptors (Schröder B et al. (1993) J Biol Chem 269, 10407-10416), from which it acts synergistically with acetylcholine (or choline) to facilitate nAChR activation (Maelicke A & Albuquerque EX (1996) Drug Discovery Today 1, 53-59; Maelicke A & Albuquerque EX (2000) Eur J Pharmacol 393, 165-170). Compounds acting like Galanthamine in this way are referred to as "allostericaly potentiating ligands (APL)" (Schrattenholz A et al. (1996) Mol Pharmacol 49, 1-6, Maelicke A & Albuquerque EX (2000) Eur J Pharmacol 393, 165-170).

The APL action on human nicotinic receptors has been demonstrated by electrophysiological studies using human brain slices (Alkondon, M. et al., (2000) J Neurosci 20, 66-75) and human recombinant cell lines each expressing a single nAChR subtype (Samochocki M et al (2000) Acta Neuro Scand Suppl 176, 68-73, Samochocki M et al. (2003) J Pharmacol Exp Therap 305, 1024-1036). All human nAChR subtypes analysed so far are sensitive to enhancement by APL. In the presence of Galanthamine, the binding affinity and channel opening probability of nAChR are increased, leading to a decrease in EC₅₀ for ACh between 30% and 65% (Samochocki M et al (2000) Acta Neuro Scand Suppl 176, 69-73, Samochocki M et al. (2003) J Pharmacol Exp Therap 305, 1024-1036). Furthermore, Galanthamine increases the slope of the dose-response curve for ACh, which has been interpreted as an increase in the cooperativity between nAChR subunits (Maelicke A & Albuquerque EX (1996) Drug Discovery Today 1, 53-59).

The APL effect of Galanthamine is observed at submicromolar concentrations (Samochocki M et al (2000) Acta Neuro Scand Suppl 176, 68-73, Samochocki M et al. (2003) J Pharmacol Exp Therap 305, 1024-1036), i.e. below the concentration range at which AChE inhibitions takes place. The two modes of action of nicotinic APL are independent of each other, as was shown by ion flux studies (Okonjo K et al (1991) Eur J Biochem 200, 671-677; Kuhlmann J et al (1991) FEBS Lett 279, 216-218) and electrophysiological studies of brain slices from both rats and humans (Santos MD et al (2002) mol Pharmacol 61, 1222-1234). In these studies, when cholinesterase activity was completely blocked by either reversible or irreversible blocking agents, the nicotinic APL, e.g. Galanthamine, still was able to produce an APL effect of the same size as in the absence of the other ChE inhibitors. Of the cholinesterase inhibitors presently approved as AD drugs, Galanthamine is the only one with nicotinic APL activity (Maelicke A et al (2000) Behav Brain Res 113, 199-206).

The use of Galanthamine and other APL as a drug treatment strategy for cognitive disorders, including AD and PD was proposed in 1996 (Maelicke A & Albuquerque EX (1996) Drug Discovery Today 1, 53-59), Later, the proposal was extended to vascular and mixed dementia (Maelicke A et al (2001) Biol Psychiatry 49, 279-288), schizophrenia, epilepsy and other diseases with a nicotinic cholinergic deficit.

The comparatively low levels of accumulation of Galanthamine in the brain are a serious disadvantage with respect to the drug's therapeutic use, i.e, for the treatment of cognitive disorders, such as AD. As indicated by the T/P ratios, only a small part of the administered drug reaches the brain, and the high levels of the drug in other (peripheral) tissues may be responsible for some of the observed adverse side effects. As a point in case, long before having been approved for the treatment of AD, Galanthamine has primarily been used for the treatment of a number of neuromuscular disorders.

EP-A 648 771, EP-A 649 846 and EP-A 653 427 all describe Galanthamine derivatives, a process for their preparation and their use as medicaments, however none of these applications considers ways and means of enhancing penetration through the blood-brain barrier and brain-to-plasma ratio of base compounds and derivatives.

US 6,150,354 refers to several Galanthamine analogues for the treatment of Alzheimer's disease. However, selective chemical modification for the purpose of increasing penetration through the blood-brain barrier is not considered.

WO 01/74920, WO 00/32199 and WO 2005030333 refer to derivatives and analogues of Galanthamine for the treatment of a variety of human brain and other diseases, and acute functional brain damage. However, selective chemical modifications or other means of improving blood-brain barrier penetration are not considered.

WO 88/08708, WO 99/21561, WO 01/43697 and US 2003/0162770 refer to derivatives and analogues of Galanthamine for the treatment of various cognitive symptoms. However, selective chemical modifications or other means of improving blood-brain barrier penetration are not considered.

WO 2005/030713 refers to a method for the synthesis of optical isomers of Galanthamine from a Narwedine bromoamide derivative. However, it does not deal with other derivatives of Galanthamine, or their use as medicaments, or chemical modifications aimed at enhancing blood-barrier penetration of said compounds.

WO 97/40049 describes several derivatives of benzazepines and related compounds that may be applied for the treatment of Alzheimer's disease. However, no concept is provided in this application for increasing the penetration of compounds through the blood-brain barrier.

The object of the present invention is the provision of procedures for achieving a favourable distribution ratio of brain to periphery for antidementia drugs of various kinds, including cholinergic receptor sensitising agents, cholinesterase inhibitors and neuroprotective drugs.

This object is met by a method and compounds as provided in the claims.

In this way the therapeutic effect-to-dose ratio can be increased and adverse side-effects can be reduced when the drugs are administered as medicaments for the diseases mentioned in the present application. This object is particularly met e.g. by site-specific chemical modification (derivatisation) of said compounds.

The present invention relates to significant enhancement in the brain-to-plasma ratio of cholinergic receptor sensitising agents, such as the APL Galanthamine (and related compounds), which is achieved by administering not the drug itself but a "pro-drug" that is converted (back) to the drug itself inside the brain of the patient. As another means for improving penetration through the blood-brain barrier (BBB) and thereby the therapeutic efficacy of the drug, the compounds themselves have been chemically modified so as to not only having larger efficacy as nicotinic APL and/or as neuroprotective agent, but in addition having enhanced lipophilicity (higher logP) or otherwise improved BBB transport properties. Due to these improvements, the pro-drugs and other compounds addressed in this application are significantly more efficacious as medicaments for the treatment of cognitive disorders than is, for example, Galanthamine. The invention applies to the compounds, selected pro-drugs and pharmaceutically acceptable salts thereof, which might be administrated via the mouth, blood, skin, by nasal application, or any other suitable application route.

Herein the term "pro-drug" refers to a derivative of a base compound wherein the group(s) added or replaced on said base compound are cleaved or returned to the group originally contained in the base compound when the derivative has reached the area or site of action. Thus, in case of a "pro-drug", an effective agent is administrated as a derivative (which is said pro-drug), however, the compound mainly or exclusively effective at the target site within the brain is the agent itself, not the derivatised compound or metabolites thereof.

The term "derivative" refers to any change of a base compound defined in the present application. The term "derivative" is used to describe a compound which either can be a pro-drug, or can be an effective agent itself/in its own right or in the derivatised form.

The terms "sensitising agent" and "allosterically potentiating ligand, APL" refer to effectors that enhance cholinergic neurotransmission by direct interaction with cholinergic receptors.

The terms "cholinergic enhancer" and "cholinergic agent" refer to compounds that enhance/modulate cholinergic neurotransmission by inhibition of cholinesterases, by allosteric sensitisation and/or direct activation of cholinergic receptors and/or by activating/modulating relevant intracellular pathways via second messenger cascades.

A derivative or pro-drug has an "enhanced blood-brain barrier permeability "according to the present invention or an "enhanced blood-brain barrier penetration" if, after administration of a pro-drug or derivative thereof to a living organism, a higher amount of said compound penetrates through the BBB, resulting in a higher level of effective agent in the brain, as compared to administration of the base compound without derivatisation. The enhanced BBB penetration should result in an increased brain-to-tissue ratio of the effective agent compared to the ratio of the base compound. Methods for determination of an enhanced BBB permeability are disclosed in this application (see supra).

The "base compound" according to the present invention preferably is Galanthamine, Norgalanthamine, Narwedine, N-Demethylnarwedine, Lycoramine, Lycoraminone, Sanguinine, Norsanguinine, and others (see table 1).

"logP" is defined as the decadic logarithm of the partition coefficient P which is the ratio of the concentration of a compound in aqueous phase to the concentration of a compound in immiscible solvent, as the neutral molecule.

The term "alkyl" shall mean a straight, branched or cyclic alkyl group of the stated number of carbon atoms. Examples include, but are not limited to methyl, ethyl, n-propyl, isopropyl, n-butyl, isobutyl, sec-butyl, t-butyl, and straight and branched chain pentyl, hexyl, heptyl, octyl, nonyl, decyl, undecyl, dodecyl, pentadecyl etc...or the according cyclic alkyls.

The term "halo" shall mean chloro, fluoro, bromo and iodo.

The term "aryl" shall mean phenyl having 0, 1, 2 or 3 substituents independently selected from the group of alkyl, alkoxy, alkylcarbonyl, halo- or trihalomethyl.

The term "cycloalkyl" shall mean a cycloalkyl group of from 3 to 12 carbon atoms and including multiple ring alkyls such as for example, adamantyl, camphoryl, and 3-noradamantyl.

In any case when a range between two limits is described it is meant that any value or integer in this range is disclosed. For example "C₁-C₈" means C₁, C₂, C₃, C₄, C₅, C₆ C₇ or Cs; or "between 0,1 and 1" means 0,1, 0,2, 0,3, 0,4, 0,5, 0,6, 0,7, 0,8, 0,9 or 1.

A "natural amino acid" is any amino acid naturally occurring in biochemical pathways or in peptides/proteins. These are particularly alanine, asparagine, cysteine, glutamine, phenylalanine, glycine, histidine, isoleucine, methionine, proline, glutamate, arginine, serine, threonine, valine, thryptophane, tyrosine, their methylated forms or the according salts.

With "sugar" is meant any suitable sugar, either an aldose or ketose, a pyranose or furanose, heptose or hexose, mono- or polysaccharide, like e.g. glucuronic acid, glucose, fructose, galactose, mannose, saccharose, lactose, maltose etc., however, glucuronic acid is preferred.

The main focus of the present invention is to improve blood-brain barrier permeability, by increasing the lipophilicity or the transport properties, or the ability of passing the blood-brain barrier, of compounds that are known to act as effective agents in correcting a cholinergic deficit, e.g. APL of nicotinic receptors or inhibitors of cholinesterases.

In one preferred embodiment the present invention refers to a method for increasing blood-brain barrier penetration of a cholinergic enhancer by preparing derivatives (either formally by their chemical structure or directly by chemical synthesis) of a molecule with a base structure of the general formula (I): 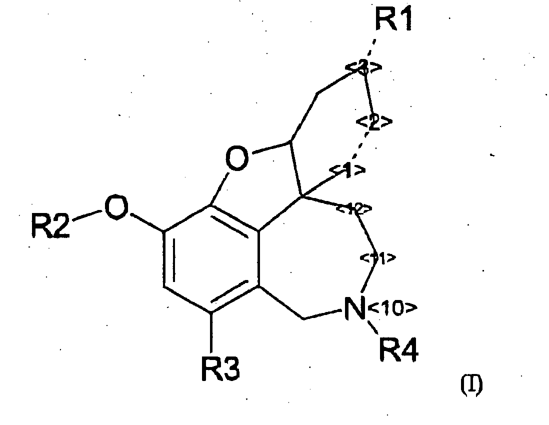 wherein the bond between positions <1> and <2> as well as <11> and <12> denotes a single- or double bond, and the bond between <10> and <11> is either a single bond or no bond.
R1 = =O, =NOH, =NH-NHCH₃, -OH, -OCOCH₃, -NH₂, or a (substituted) derivative of the ketone, like semicarbazone, thiosemicarbazone, aminoguanidine etc.
R2 = H, CH₃, acetyl
R3 = H, CH₃, F, Cl, Br, I
R4 = H, CH₃

In table I, compounds are exemplified with a base structure of the general formula (II) 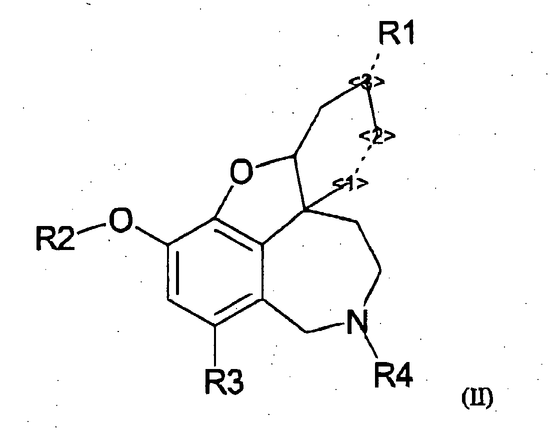 that belong to the structures summarised in formula (I):

**Table 1:**

| R1 | R2 | R3 | R4 | Bond <1>-<2> | Bond <3>-R1 | Name | logP calcd.(1) |
|---|---|---|---|---|---|---|---|
| OH | CH₃ | H | CH₃ | Double | Single | Galanthamine | 1.30 |
| OH | CH₃ | H | H | Double | Single | Norgalanthamine | 1.38 |
| OH | H | H | CH₃ | Double | Single | Sanguinine | 0.83 |
| OH | H | H | H | Double | Single | Norsanguinine | 0.91 |
| MeCH(OH) CH₂-CO | H | H | CH₃ | Double | Single | Leucotamine | 1.23 |
| OH | CH₃ | H | CH₃ | Single | Single | Lycoramine | 1.28 |
| OH | CH₃ | H | H | Single | Single | Norlycoramine | 1.36 |
| O | CH₃ | H | CH₃ | Single | Double | Lycoraminone | 0.85 |
| O | CH₃ | H | CH₃ | Double | Double | Narwedine | 0.74 |
| O | CH₃ | H | H | Double | Double | Nornarwedine | 0.82 |
| NH2 | CH₃ | H | CH₃ | Double | Single | 3-Amino-3-deoxy-galanthamine | 1.05 |
| NH2 | CH₃ | H | CH₃ | Single | Single | 3-amino-3-deoxy-1,2-dihydro-galanthamine | 0.89 |

| | | | | | | | |
|---|---|---|---|---|---|---|---|
| (1) Calculated using Advanced Pharma Algorithms Software ToxBoxes V1.0.2 | | | | | | | |

The compounds listed in table 1, and other compounds to be used as a base compound for derivatisation according to the present invention, can be obtained either by isolation from natural sources or by total chemical synthesis, or by chemical modification of natural or synthetic compounds.

The compounds to be used according to the present invention can be derivatives of the above listed molecules that can be demonstrated to act as cholinergic enhancers. This property of said derivatives may be manifested by one or more of the following properties; by their ability to sensitise cholinergic receptors, and/or inhibit brain cholinesterases, and/or modulate intracellular messenger levels, and/or act neuroprotective. The ability to act as sensitising agent on nicotinic receptors can be determined by electrophysiological and Ca-imaging methods, as described in Schrattenholz A et al. (1996) Mol Pharmacol 49, 1-6 and Samochocki M et al (2000) Acta Neuro Scand Suppl 176, 68-73; Samochocki M et al. (2003) J Pharmacol Exp Therap 305, 1024-1036. The ability to inhibit cholinesterases can be determined by the photometric method of Ellman et al., Biochem. Pharmacol. 7,88 (1961). The ability to modulate intracellular messenger levels can be determined by Ca-imaging methods (Samochocki M et al. (2003) J Pharmacol Exp Therap 305, 1024-1036) and other means of recording changes in intracellular messenger levels or effects resulting thereof (Kihara T et al (2004) Biochem Biophys Res Commun 325, 976-982). The ability to act neuroprotective can be determined by a variety of in vitro and in vivo test systems, including in cell culture (Arias E et al (2003) Neuropharmacol 46, 103-1S 14; Kihara T et al (2004) Biochem Biophys Res Commun 325, 976-982) and in animal models of neurodegenerative diseases (Capsoni et al (2002) Proc Natl Acad Sci USA 99, 12432-12437).

As specific examples, table 2 exemplifies compounds that are derivatives of a base structure of the following general formula (M) 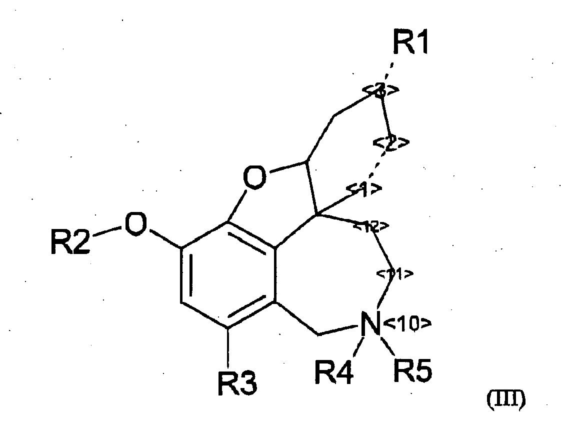 and act in any way as cholinergic enhancers:

**Table 2:**

| R1 | R2 | R3 | R4 | R5 | Bond 1-2 | Bond 3-R1 | Bond 10-11 | Bond 11-12 | Name | logP calcd(1) |
|---|---|---|---|---|---|---|---|---|---|---|
| OH | CH₃ | H | CH₃ | CH₃ | D | S | N | S | 10,11-Seco-10-methyl-galanthamine | 2.67 |
| OH | CH₃ | H | CH₃ | H | D | S | N | D | 10,11-Seco-11,12-dehydro-galanthamine | 2.09 |
| NOH | CH₃ | H | CH₃ | e | D | D | S | S | Narwedinoxim | 1.15 |
| NNHCH₃ | CH₃ | H | CH₃ | e | D | D | S | S | Narwedin-N-methyl-hydrazone | 0.34 |
| OH | CH₃ | F | CH₃ | e | D | S | S | S | 8-Fluoro-galanthamine | 1.25 |
| OH | CH₃ | Br | CH₃ | e | D | S | S | S | 8-Bromo-galanthamine | 2.27 |
| OH | CH₃ | I | CH₃ | e | D | S | S | S | 8-Iodo-galanthamine | 2.26 |
| OH | CH₃ | Br | CH₃ | O | D | S | S | S | 8-Bromo-galanthamine-N-oxide | 2.68 |

| | | | | | | | | | | |
|---|---|---|---|---|---|---|---|---|---|---|
| (1) Calculated using Advanced Pharma Algorithms Software ToxBoxes V1.0.2. Abbreviations: s: single bond; d: double bond; n: no bond; e: electron pair | | | | | | | | | | |

Most of the compounds listed in table 2 are not only efficacious agents in one or more of the tests cited above, but most of them also have more favourable logP and transport properties than the base compounds from which they are derived.

To further improve BBB permeability and brain/plasma distribution ratio, modifications of the following kinds can be performed so as to make the compounds exemplified in tables 1 and 2 more lipophilic or enhance otherwise their transport into the CNS, in comparison to the base compound:
1. Conjugations to groups or molecules that are known to occur in the course of metabolic conversions, e.g. carbohydrate conjugates such as glycosyls, glucuronides and natural metabolites, or are otherwise known to readily pass the blood-brain barrier, e.g. aminoacids, vitamins, various messenger molecules and drugs.
2. Conjugations to groups leading to quaternary ammonium salts with a labile nitrogen-carbon bond (see e.g. example 1).
3. Conjugations to groups leading to esters, e.g. acylderivates with enhanced lipophilicity and BBB penetration properties. For example, such compounds may be esters of the oxygen function in position 3 and/or 6 of the following base structure (IV): 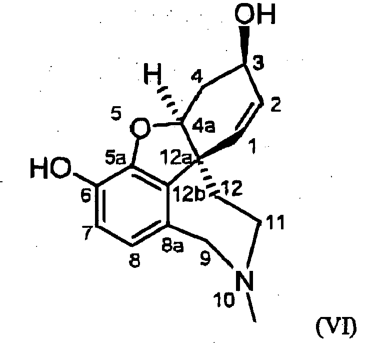
   a) Esters with saturated or unsaturated fatty acids containing 1-22 carbon atoms optionally containing an additional (ar)alkoxy or di(ar)alkylamino group
   b) Esters with carbonic acid where one acidic function of carbonic acid is esterified with the 3- and/or 6-position of galanthamine and the other represents an ester as defined in 3 a.
   c) Esters with (substituted) pyridine- or (substituted) dihydropyridine-carboxylic acids (see e.g. example 2)
4. Formation of ketals or aminals of substituents in positions 3, 6, and 10 that increase the lipophilicity and are hydrolyzed to the desired derivatives, e.g. (nor)galanthamine derivatives (see e.g. examples 3 and 4)
5. Formation of basic and/or quaternary carbamates of said compounds that are chemically or metabolically unstable.
6. Conjugation to a lipophilic dihydropyridinium carrier, e.g. as 1,4-dihydro-1-methyl-3-pyridinecarboxylate, that in the brain is enzymatically oxidised to the corresponding ionic pyrimidinium salt.
7. Conjugation with nicotinic acid, nicotinic acid amide, various cofactors, messenger molecules and other chemical entities that enhance lipophilicity and transport through the BBB.

These modifications lead to compounds of the following general formula (III) 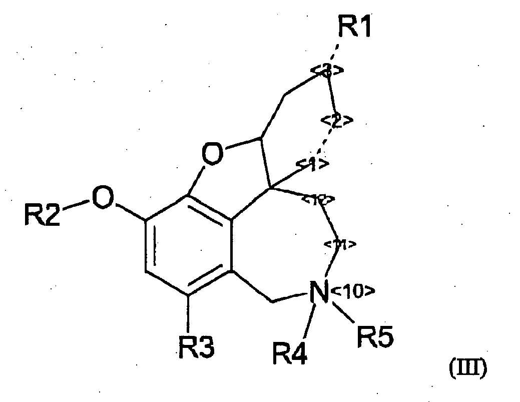 wherein the bond between positions <1> and <2> denotes a single- or double bond, with the proviso that the structure is not any of those listed in Table 1 and the bonds <1> to <2> and <11> to <12> can be either a single or a double bond, and the bond between <10> and <11> is either a single bond or no bond and the residues R1 - R5 are defined as follows:
R1:
   a) if bond <3> to R1 is a double bond, then R1 = O, NH, NOH, NOR6, N-CO-NH₂, N-CS-NH₂, N-C(=NH)-NH₂, N-NH-phenyl, N-NHR₆, N-N(R6)₂, N-N=(CH₂)ₙ with R6 = C₁-C₅ unbranched or branched, saturated or unsaturated (ar)alkyl, phenyl or benzyl and n=2-8
   b) if bond <3> to R1 is a single bond, then R1 = OH, SH, NH₂, NHR6, N(R6)₂, OR7, O-CR8R9-O-CO-CHR10-NR11R12
      with R7 = C₁-C₂₂ unbranched or branched, (poly-)unsaturated or saturated alkyl, optionally containing an additional (ar)alkoxy or di(ar)alkylamino group, a sugar or sugar derivative residue, preferably glucuronic acid, or COR13,
      where
      R13 = R6 or R7 or pyridyl or dihydropyridyl or OR6, preferably methyl, 3-pyridyl, 4-pyridyl, 3-dihydropyridyl, 4-dihydropyridyl R8 and R9 are the same or different and any of H, Me, Ph or they together form spiro-ring -(CH₂)ₙ- with n=4-6
      R10 = H or the side chain of a natural amino acid including R10 and
      R11 together are forming a proline or hydroxy-proline derivative
      R11 either is together with R10 forming a proline or hydroxy-proline derivative or is H
      R12 is a carbamate protecting group including t-butoxycarbonyl, benzyloxycarbonyl and other N-protecting groups
R2:
   H, R7, or O-CR8R9-O-CO-CHR10-NR11R12, with the same definitions of R7-R12 as above
R3:
   H, F, Cl, Br, I, NH₂, NO₂, CN, CH₃
R4:
   H or CH₃
R5:
   If R4 = H**,** then R5 is an electron pair
   ifR4 = CH₃ then R5 is either hydrogen or a C₁-C₅ (ar)alkyl group, CH₂-O-CH₃,
   CH₂-O-, CO-R6, CH₂-O-CR8R9-O-CO-CHR10-NR11R12 with the same definitions of R6 and R8-R12 as above;
   in all the latter cases the nitrogen bears an additional positive charge as well as a counterion, selected from chloride, bromide, iodide, sulphate, nitrate, hydrogensulfate, phosphate, methanesulphonate, tosylate or other pharmaceutically acceptable anion.

Preferred derivatives of the main concept of the invention are quarternary ammonium salts with a labile nitrogen-carbon bond at R5; mono- or diacylderivatives (esters) of the hydroxyl groups of said base compounds (R1, R2); sugar derivatives, preferably glucuronides (R1, R2); derivatives coupled with nicotinic acid (R1, R2); and selected halogenides (R3).

Another preferred derivative of the main concept is a lipophilic dihydropyridinium carrier. This Redox Chemical Delivery System (RCDS; Misra A et al (2003) J Pharm Pharmaceut Sci 6, 252-273) is known to significantly enhance drug delivery through the BBB into the brain parenchyma. Once inside the brain, the dihydropyridinium moiety is enzymatically oxidized to the corresponding ionic pyridinium salt. Subsequent cleavage of the original compound from the carrier leads to liberation of the original compound and to sustained levels of it in the brain tissue.

Other preferred derivatives of the main concept are aminoacids that are known to be transported into the brain by active aminoacid carriers, e.g. tyrosine. Once inside the brain parenchyma, these derivatives can either directly act on their target molecules or are first enzymatically liberated before acting as the original aren't compound.

As a further aspect of the present invention, the derivatives obtained by chemical modification do not need to work as such as medicaments but rather may initially be pro-drugs that, after penetration though the blood-brain barrier, are converted (e.g. by brain enzymes) to the parent compound or a metabolite thereof and work as such as a medicament. Said pro-drug or derivative is used to prepare a medicament or pharmaceutical composition that preferably can be used for the treatment of brain diseases associated with a cholinergic deficit.

Of the derivatives contained in the general structure of formula (III) and with the proviso and definitions provided there, the following are of particular interest in regard to the present invention, as they have not yet been described or developed under the premise of having higher lipophilicity and/or better BBB transport properties and/or higher brain-to-plasma ratio than their parent compounds (table I) from which they are derived by chemical modification:

Table 3: Examples of compounds described in previous publications/patents presently shown that they (i) act as cholinergic enhancers, and/or (ii) have higher logP-values than Galanthamine

| STRUCTURE | logP | Name |
|---|---|---|
| 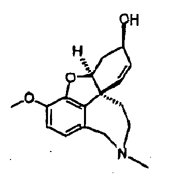 | 1.30 | Galanthamine |
| 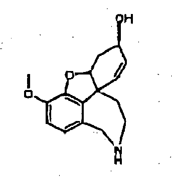 | 1.38 | Norgalanthamine |
| 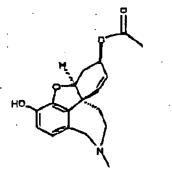 | 1.68 | 3-O-Acetyl-6-O-demethyl-galanthamine |
| 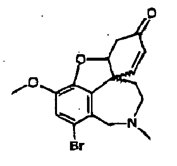 | 1.72 | 8-Biomonarwedine |
| 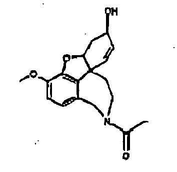 | 1.76 | Narcisine |
| 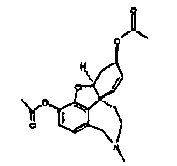 | 1.99 | |
| 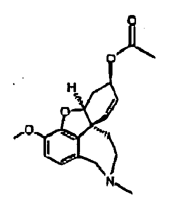 | 2.15 | |
| 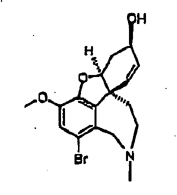 | 2.27 | |
| 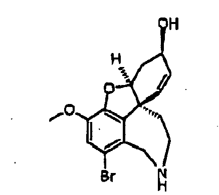 | 2.35 | |
| 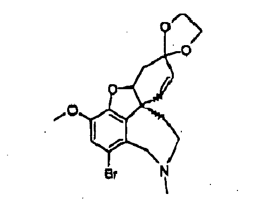 | 2.69 | |
| 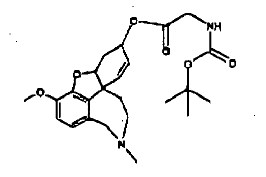 | 3.07 | |
| 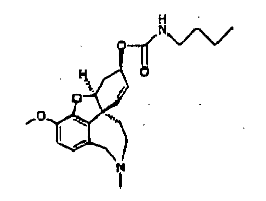 | 3.27 | |
| 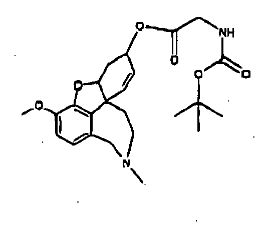 | 4.09 | |
| 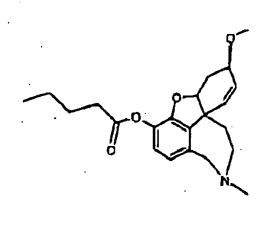 | 4.90 | |

Lit1: Han, So Yeop; Maycr, Scott C.; Schweiger, Edwin J.; Davis, Bonnie M.; Joullie, Madeleine M. Synthesis and biological activity of galanthamine derivatives as acetylcholinesterase (AChE) inhibitors. Bioorganic & Medicinal Chemistry Letters (1991), 1(11), 579-80.

The following derivatives covered by the general structure of formula (III) and with the proviso and definitions provided there are particularly preferred derivatives of the main concept of the invention in that they have not yet been mentioned or described in any other publication or patent.

Table 4: Examples of new compounds that (i) act as cholinergic enhancers, and/or (ii) have higher logP-values than Galanthamine (the latter being included in the table for comparison only)

| No. | STRUCTURE | logP | Example Number |
|---|---|---|---|
| 1 | 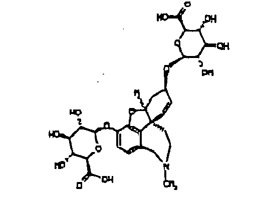 | -3.08 | 8 |
| 2 | 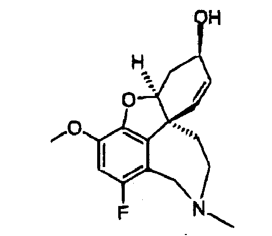 | 1.25 | 10 |
| 3 | 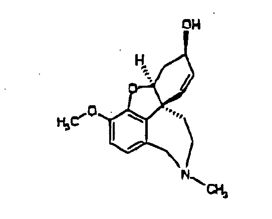 | 1.30 | Galanthamine mentioned here just for comparison! |
| 4 | 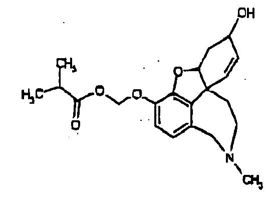 | 1.57 | |
| 5 | 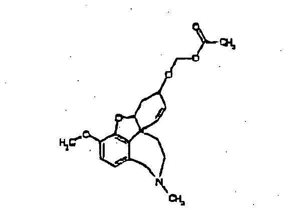 | 1.63 | |
| 6 | 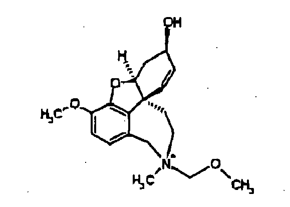 | 1.64 | 1 |
| 7 | 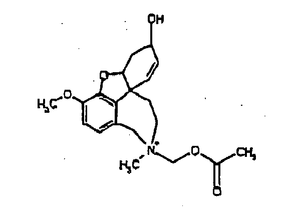 | 1.68 | |
| 8 | 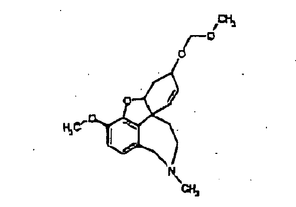 | 1.72 | |
| 9 | 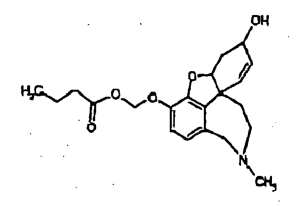 | 1.80 | |
| 10 | 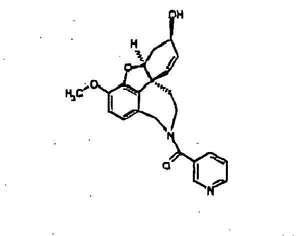 | 1.82 | |
| 11 | 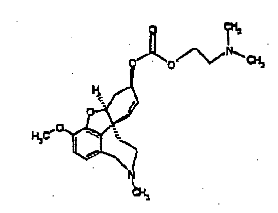 | 1.87 | |
| 12 | 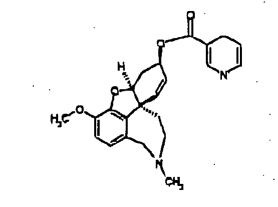 | 1.87 | |
| 13 | 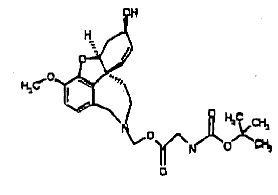 | 1.96 | 2 |
| 14 | 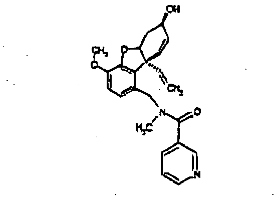 | 2.04 | |
| 15 | 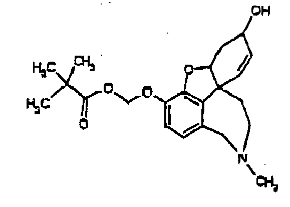 | 2.05 | |
| 16 | 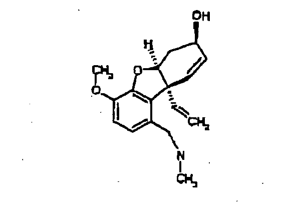 | 2.09 | 5 |
| 17 | 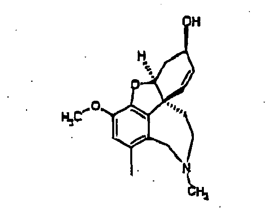 | 2.26 | |
| 18 | 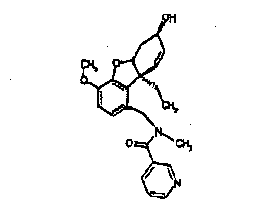 | 2.27 | |
| 19 | 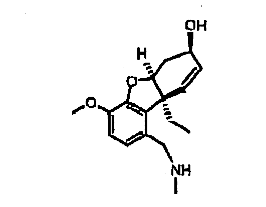 | 2.33 | 7 |
| 20 | 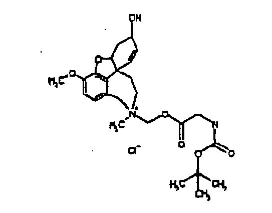 | 2.37 | |
| 21 | 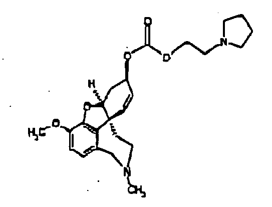 | 2.39 | |
| 22 | 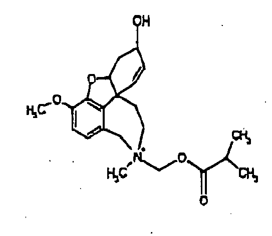 | 2.42 | |
| 23 | 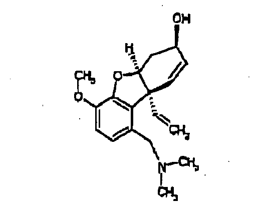 | 2.44 | 4 |
| 24 | 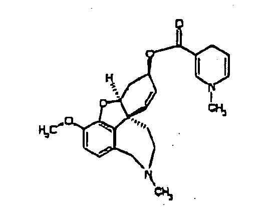 | 2.45 | |
| 25 | 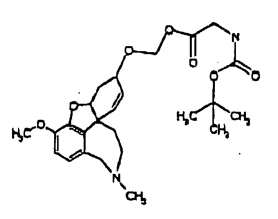 | 2.55 | |
| 26 | 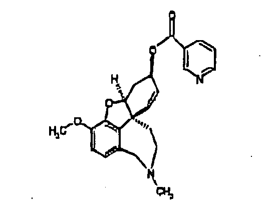 | 2.62 | 9 |
| 27 | 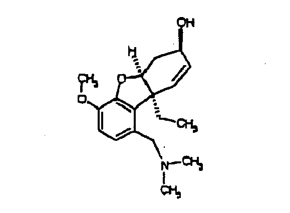 | 2.66 | 6 |
| 28 | 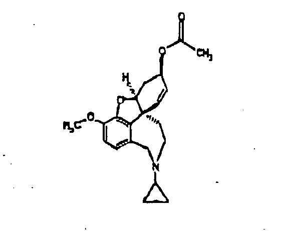 | 2.81 | |
| 29 | 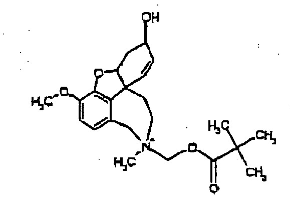 | 2.90 | |
| 30 | 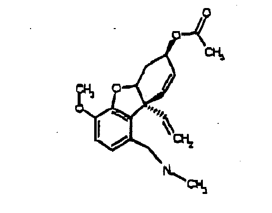 | 294 | |
| 31 | 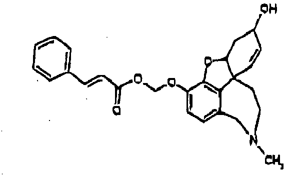 | 3.15 | |
| 32 | 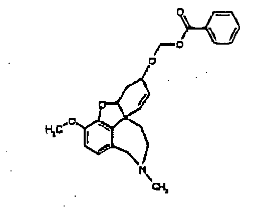 | 3.31 | |
| 33 | 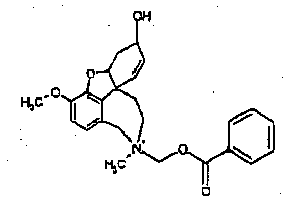 | 3.36 | |
| 34 | 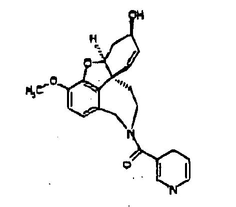 | 3.66 | |
| 35 | 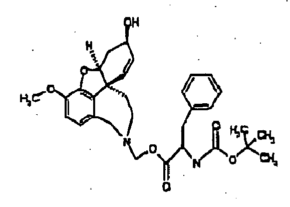 | 3.67 | 3 |
| 36 | 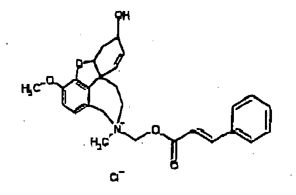 | 3.69 | |
| 37 | 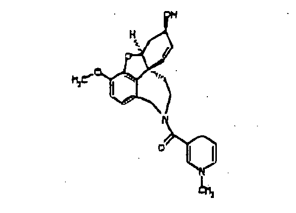 | 3.93 | |
| 38 | 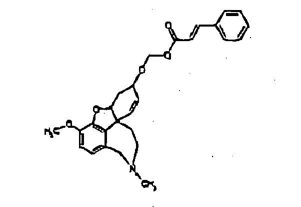 | 3.95 | |
| 39 | 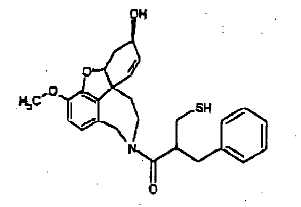 | 3.99 | |
| 40 | 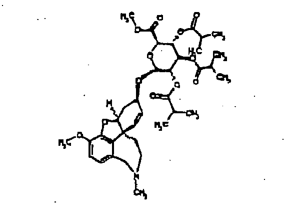 | 4.07 | 8 |
| 41 | 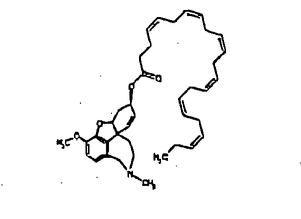 | 10.61 | |

The derivative shown in tables 3 and 4 may be used to prepare a medicament or other pharmaceutical composition. Such medicament or pharmaceutical composition can be used for the treatment of a disease state associated with a cholinergic deficit.

The usefulness of the derivatives, before and/or after conversion to the parent compound, to act as effective pharmaceutical agents is manifested by their ability to sensitise cholinergic receptors, and/or inhibit brain cholinesterases, and/or modulate intracellular messenger levels, and/or act neuroprotective. The ability to act as sensitising agent on nicotinic receptors can be determined by electrophysiological and Ca-imaging methods, as described in Schrattenholz A et al. (1996) Mol Pharmacol 49, 1-6 and Samochocki M et al (2000) Acta Neuro Scand Suppl 176, 68-73; Samochocki M et al. (2003) J Pharmacol Exp Therap 305, 1024-1036. The ability to inhibit cholinesterases can be determined by the photometric method of Ellman et al., Biochem. Pharmacol. 7,88 (1961). The ability to modulate intracellular messenger levels can be determined by Ca-imaging methods (Samochocki M et al. (2003) J Pharmacol Exp Therap 305, 1024-1036) and other means of recording changes in intracellular messenger levels or effects resulting thereof (Kihara T et al (2004) Biochem Biophys Res Commun 325, 976-982). The ability to act neuroprotective can be determined by a variety of in vitro and in vivo test systems, including in cell culture (Arias E et al (2003) Neuropharmacol 46, 103-1S 14; Kihara T et al (2004) Biochem Biophys Res Commun 325, 976-982) and in animal models of neurodegenerative diseases (Capsoni et al (2002) Proc Natl Acad Sci USA 99, 12432-12437).

This usefulness can also be ascertained by determining the ability of these compounds (1) to reduce neuronal cell death and amyloid plaque formation as well as cognitive impairment in animal models of Alzheimer's disease (Capsoni et al (2002) Proc Natl Acad Sci USA 99, 12432-12437) and (2) to enhance learning performance in various animal test systems. In one particular learning paradigm applied to old and young rabbits (Woodruff-Pak D et al (2001) Proc Natl Acad Sci USA, 98, 2089-2094), the classical eye blink conditioning is used to study the effect of cognition-enhancing drugs on the septohippocampal cholinergic system. An active test compound of the present invention will reduce the number of trials required to learn that the air blow applied onto the animal's eye does not require the animal to close the eye (eye blink) as a protective measure.

This usefulness can also be ascertained by determining the ability of these compounds to restore deficient memory due to a cholinergic deficit in the Dark Avoidance Assay (DAA). In this assay mice are tested for their ability to remember an unpleasant stimulus for a period of e.g. 24 hours. A mouse is placed in a chamber that contains a dark compartment; a strong incandescent light drives it to the dark compartment, where an electric shock is administered through metal plates on the floor. The animal is removed from the testing apparatus and tested again, 24 hours later, for the ability to remember the electric shock administered in the dark compartment.

If a nicotinic or muscarinic antagonist, i.e. an anticholinergic drug that causes memory impairment, is administered before an animal's initial exposure to the test chamber, the animal tends to re-enter the dark compartment much sooner than in the absence of the anticholinergic drug when being placed in the test chamber 24 hours later. This effect of an anticholinergic drug is blocked by an active test compound, resulting in a greater interval before re-entry into the dark compartment.

The test results may be expressed as the percent of a group of animals in which the effect of the anticholinergic drug is blocked or reduced, as manifested by an increased time interval between being placed in the test chamber and re-entering the dark compartment.

According to the present intention and approach, the brain disease that can be treated with the pro-drugs and derivatives provided herewith can be any psychiatric, neurological and neurodegenerative disease associated with a cholinergic deficit of any kind, including a neurodegenerative loss of cholinergic neurotransmitters and/or receptors, ACh-synthesising and metabolising enzymes, transport proteins and the like. Such diseases are exemplified by Alzheimer's and Parkinson's disease, other types of dementia, schizophrenia, epilepsy, stroke, poliomyelitis, neuritis, myopathy, oxygen and nutrient deficiencies in the brain after hypoxia, anoxia, asphyxia, cardiac arrest, chronic fatique syndrome, various types of poisoning, anesthesia, particularly neuroleptic anesthesia, spinal cord disorders, inflammation, particularly central inflammatory disorders, postoperative delirium and/or subsyndronal postoperative delirium, neuropathic pain, subsequences of the abuse of alcohol and drugs, addictive alcohol and nicotine craving, and subsequences of radiotherapy, and more. The effect of Galanthamine or other cholinesterase inhibitors in treatment of such diseases are described e.g. in WO2005/74535, WO2005/72713, WO2005/41979,WO2005/30332, WO2005/27975, US2004/266659 and W02004/14393.

All the derivatives described in the general (base) structure of formula (III) and tables 2, 3 and 4 either have an effect as a pro-drug, which means that the derivative, after entering the brain, is "converted back" into an effective agent, e.g. Galanthamine, Narwedine, Lycoramine, or the other said base compounds, or they are effective (i.e. as cholinergic enhancers or agents according to the definition) as derivatives themselves, meaning that they are not necessarily converted or metabolised before they act as agents at their target molecules, e.g. cholinergic receptors or cholinesterases. The common feature of the derivatives of the present application is that they all penetrate more effectively through the blood-brain barrier than the base compound, which according to the present invention preferably is Galanthamine and related compounds. As a result of their improved BBB penetration properties, these compounds should have higher therapeutic efficacy and lower adverse side effects than e.g. Galanthamine.

The compounds of the present invention whether pro-drugs or otherwise effective agents can be administered as such or as a pharmaceutically acceptable salt thereof.

The derivatives of the common formulae as defined above can be prepared by any known method, however, it is preferred that the derivatives are prepared with proper use by the methods described for derivatisation of according compounds in EP-A 649 846 with reference to scheme I and in the examples; EP-A648 771 with reference to scheme I and in the examples; EP-A 653 427 with reference to scheme I and in the examples; US 6,150,354, paragraph "procedures" and examples; or US 6,638,925, paragraph "experimental section", respectively. A further reference is WO 01/74820, wherein combinatory and/or parallel synthesis is disclosed and synthesis of several compounds is described in the examples. Further the method can be used as described in Gomes, P.et al., Rui. Centro de Investigacao em Quirnica da Universidade do Porto, Oporto, Port. Synthetic Communications (2003), 33(10), 1683-1693. A skilled person clearly will understand that in any case an appropriate educt/ appropriate educts has/have to be used to obtain the desired derivatisation of the base structure. The preparation method is not limiting the invention as long as the compounds presently described are obtained.

The compounds of the invention preferably are prepared from the appropriate optical isomer of Galanthamine or Narwedine via the intermediate 6-demethylgalanthamine, a known therapeutically effective compound, or 6-demethylnarwedine, respectively.

The pro-drugs and derivatives of this invention are selected by the following tests, which shall be considered as examples not limiting the invention:
1. Activity as nicotinic "allosterically potentiating ligand (APL), preferentially determined by electrophysiological methods and, Ca-imaging, using human cell lines that express individual subtypes of human neuronal nicotinic acetylcholine receptors (nAChR).
   - In the case of a compound acting as such: The activation of nAChR by ACh or agonist is enhanced in the presence of said compound, with the APL activity being selectively blocked by antibody FK1.
   - In the case of a pro-drug: Enhanced activity as a centrally acting APL after the pro-drug has been converted to the base compound by treatment with a rat brain or human brain homogenate extract.
   - Kinetics of conversion from pro-drug to drug when incubated with a rat or human brain extract.
2. Activity as centrally acting cholinesterase inhibitor, as tested by various in-vitro, cell culture and in-vivo test systems.
   - In the case of a pro-drug: Enhanced cholinesterase inhibition - or the same level of inhibition at a significantly reduced dose - is observed when the pro-drug is administered instead of the original base compound.
   - Kinetics of conversion from pro-drug to drug when incubated with a rat or human brain extract.
3. Neuroprotective activity in acute toxicity protection tests (organophosphate poisoning of animals, in-vitro poisoning by Aß and/or glutamate) and in animal models of neurodegeneraton.
   - In the case of a pro-drug: Enhanced neuroprotective activity - or the same level of neuroprotection at a significantly reduced dose - is observed when the pro-drug is administered instead of the original base compound.
4. Accumulation of the derivatives in the brain of mammals as compared to unmodified Galanthamine or other base compound.
5. Lipophilicity, as measured by shake-flask (e.g. octanol/buffer), HPLC-retention and nanobeads absorption methods.
6. Bioconversion t_{1/2} in the brain as compared to blood (systemic).
7. Theoretical/empirical estimates of distribution and log P values.
8. Other miscellaneous tests.

As one way of estimating improved lipophilicity of the derivatised compounds, logP-values are provided in some of the tables. Improved lipophilicity, as characterized by an increased logP-value, can either be determined experimentally including HPLC methods or by predictive computational methods. Although such calculations cannot replace the experiment, the data are strongly suggestive as to whether a certain modification of the base compound will result in an improved lipophilicity. Computer programs that allow such calculations include e.g. ToxBoxes from Pharma Algorithms, ACD-Lab, Molecule Evaluator from Cidrux, and others.

Another means of estimating the readiness of a compound to transverse the BBB is by experimental comparison of the membrane affinity of said compound to its binding affinity to serum albumin, both determined by the NIMBUS Biotechnology assay (Willmann, S. et al. (2005) J Med Chem, in print).

Effective quantities of the compounds of the invention may be administered to a patient by any of various methods, including orally as in capsules or tablets, via the skin or by nasal application. The free base final products, while effective by themselves, may be formulated and administered in the form of a pharmaceutically acceptable salt, e.g. for purposes of stability, convenience of crystallization, increased solubility, release retardation, and the like.

Since the pro-drugs / compounds of the present invention pass the blood-brain-barrier easier than the base compounds, there are two advantageous aspects: first is the fast uptake of the pro-drug and therefore a fast onset of effect, second is that the dosage of application can be decreased compared to known medicaments resulting in lower peripheral side effects with high efficacy of the compounds at their effect site (brain). Further the pro-drugs after passage through the blood-brain-barrier are converted in the base compound which has a lower permeability through the blood-brain-barrier, thus the effective compound remains in the brain, resulting in a longer time period of effectiveness.

As a representative case, the active compounds of the present invention may be orally administered, for example, with an inert diluent or with an edible carrier, or they may be enclosed in gelatine capsules, or they may be compressed into tablets. Furthermore, the active compounds of the invention may be incorporated with excipients and used in the form of tablets, troches, capsules, elixirs, suspensions, syrups, wafers, chewing gum and the like. Preferred compositions and preparations according to the present invention are prepared so that an oral dosage unit form contains between 0.1 and 50 milligrams of active compound.

Because BBB penetration and brain-to-plasma ratio of the compounds modified according to this invention, are significantly enhanced, the dosages of administered drug may be dramatically reduced, as compared to previous applications, clinical studies and estimates.

Acids useful for preparing the pharmaceutically acceptable acid addition salts according to the invention include inorganic acids and organic acids, such as sulfamic, amidosulfonic, 1,2-ethanedisulfonic, 2-ethylsuccinic, 2-hydroxyethanesulfonic, 3-hydroxynaphthoic, acetic, benzoic, benzenesulfonic acid, carboxylic, ethylenediamine tetraacetic acid, camphorsulfonic, citric, dodecylsulfonic, ethanesulfonic, ethenesulfonic, ethylenediamine tetraacetic, fumaric, glubionic, glucoheptonic, gluconic, glutamic, hexylresorcinic, hydrobromic, hydrochloric, isethionoc, (bi)carbonic, tartaric, hydriodic, lactic, lactobionic, laevulinic, laurylsulfuric, lipoic, malic, maleic, malonic, mandelic, methanesulfonic, mucic, naphthalenesulfonic, nitric, oxalic, pamoic, pantothenic, perchloric, phosphoric, polygalacturonic, pectic, propionic, salicylic, succinic or sulfuric acid, p-tuluenesulfonic, wherein hydrochloric, hydrobromic, sulfuric, nitric, phosphoric and perchloric acids, as well as tartaric, citric, acetic, succinic, maleic, fumaric and oxalic acids are preferred.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but may be varied depending upon the particular form and may conveniently be between 5% to about 70% of the weight of the unit. The amount of active compound in such compositions is such that a suitable dosage will be obtained. Preferred compositions and preparations according to the present invention are prepared so that an oral dosage unit form contains between 0.1 - 50 milligrams of active compound.

The tablets, pills, capsules, troches and the like may also contain the following ingredients: a binder such as micro-crystalline cellulose, gum tragacanth or gelatin: an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavouring agent such as peppermint, methyl salicylate, or orange flavouring. When the dosage unit form is a capsule, it may contain, in addition to materials of the above-type, a liquid carrier such as an oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent and certain preservatives, dyes, colourings and flavours. Materials used in preparing these various compositions should be pharmaceutically pure and non-toxic in the amounts used.

For the purpose of nasal or parenteral therapeutic administration, the active compounds of the invention may be incorporated into a solution or suspension. These preparations should contain at least 0.1 % of active compound, but may be varied between 0.5 and about 30% of the weight thereof. The amount of active compound in such compositions is such that a suitable dosage will be obtained. Preferred compositions and preparations according to the present inventions are prepared so that a nasal or parenteral dosage unit contains between 0.1 to 20 milligrams of active compound.

Further the compounds of the present invention can be administered via intranasal delivery to the cerebral spinal fluid as disclosed in detail in WO2004/0240

The solutions or suspensions may also include the following components: a sterile diluent such as water for injection, saline solution, fixed oils, polyethylene glycols, glycerine, propylene glycol or other synthetic solvents; antibacterial agents, such as benzyl alcohol or methyl parabens; antioxidants such as ascorbic acid or sodium bisulfite; chelating agents such as ethylene-diamine tetraacetic acid; buffers such as acetates; citrates or phosphates and agents for the adjustment of tonicity such as sodium chloride or dextrose. Parenteral multiple dose vials may be of glass or plastic.

Typical dosage rates in administration of the active ingredients depend on the nature of the compound that is used and in intravenous administration are in the range of 0.01 to 2.0 mg per day and per kilogram of body weight based on the physical condition and other medications of the patient.

The following specific formulations exemplify suitable applications: Tablets and capsules that contain 0.5 to 50 mg. Solution for parenteral administration that contains 0.1 to 30 mg of active ingredient/ml. Liquid formulations for oral administration at a concentration of 0.1 to 15 mg/ml. Liquid formulations for nasal or intra-cerebroventricular administration at a concentration of 0.1 to 5 mg of active ingredient/ml. The compounds according to the invention can also be administered by a transdermal system, in which 0.1 to 10 mg/day is released. A transdermal dosage system may consists of a storage layer that contains 0.1 to 30 mg of the active substance as a free base or salt, in case together with a penetration accelerator, e.g., dimethyl sulfoxide, or a carboxylic acid, e.g., octanoic acid, and a realistic-looking polyacrylate, e.g., hexylacrylate/vinyl acetate/acrylic acid copolymer including softeners, e.g., isopropylmyristate. As a covering, an active ingredient-impermeable outside layer, e.g., a metal-coated, siliconised polyethylene patch with a thickness of, for example, 0.35 mm, can be used. To produce an adhesive layer, e.g., a dimethylamino-methacrylate/methacrylate copolymer in an organic solvent can be used.

The invention also relates to pharmaceutical compositions that in a pharmaceutically acceptable adjuvant contain a therapeutically effective amount of at least one of the compounds that are proposed according to the invention.

### Examples of chemical synthesis and properties of derivatives:

Example 1: N-Methoxymuthyl-galanthaminiumchloride (= (4aS,6R,8aS)-4a,5,9,10,11,12-Hexahydro-11-methoxymethyl-11-methyl-6*H*-6-hydroxy-3-methoxy-benzofuro[3a,3,2-ef][2]benzaze-pinium, chloride).

N-Methoxymethyl-galanthaminiumchloride is obtained from Galanthamine via alkylation using chloromethylmethylether:

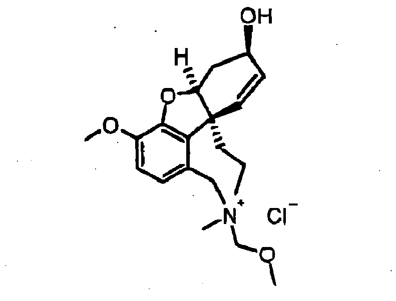

To a solution of (-)-Galanthamine (5.00 g, 17.4 mmol) in dry dimethylformamide (12 mL) chloromethylmethylether (1.12 g, 13.9 mmol) is added at -5 bis 0 °C in the course of 15 min and stirred for 4 hrs. at room temperature. The reaction mixture is poured on ethyl acetatet (500 mL) and the precipitate obtained is filtered and washed using ethyl acetate (3 x 50 mL).

The crude product (4.20 g, 82 %) has a purity of 96 % (HPLC). For further purification the crude product is dissolved in dry ethanol, stirred after the addition of activated charcoal, filtered and added to ethyl acetate (500 mL). The precipitate is filtered and washed using ethyl acetate (3 x 50 mL) and dry diethylether (1 x 50 mL). The product is obtained in the form of colourless crystals (3.85 g, 75 % d. Th.) melting at 126-127°C. Opt. Rotation:_[α]_{D}²⁰ = -113.9° (c= 0.18 g / water) calcd. For C₁₉H₂₆ClNO₄* 0.33 H₂O C, 61.04; H, 7.19; N, 3.75; found: C, 61.10; H, 7.07; N, 3.75

¹H NMR (DMSO-d₆)_δ 6.86 (s, 2H), 6.29 (d, *J*= 10 Hz, 1H), 5.88 (d, *J=* 10 Hz, *J*= 4 Hz, 1H), 5.13 (bs, 3H), 4.66 (s, 2H), 4.48 (d, *J=* 14 Hz, 1H), 4.22 - 3.90 (m, 2H), 3.81 (s, 3H), 3.70 (s, 3H), 3.70 - 3.52 (m, 1H), 2.75 (s, 3H), 2.44 - 1.79 (m, 4H); ¹³C NMR (DMSO-d₆) δ 146.4 (s), 145.2 (s), 132.8 (s), 130.2 (d), 125.3 (d), 123.7 (d), 117.8 (s), 112.1 (d), 94.8 (t), 86.4 (d), 61.7 (d), 60.3 (t), 59.4 (q), 56.2 (t), 55.6 (q), 46.2 (s), 40.2 (q), 31.1 (2 t);

The chemical and biological stability of this compound has been determined in various buffers (chemical stability), in rat blood serum, and in rat brain extract, suggesting that the derivative can act as a pro-drug.

Instead of chloromethyl or methyl ether the following reagents can be used alternatively: Methoxymethanolbenzenesulfonate, trifluoromethanesulfonic acid methoxymethyl ester, or methoxymethanol 4-methylbenzenesulfonate.

Example 2: Tert-Butoxycarbonylamino-acetic acid (N-norgalanthaminyl)-methyl ester

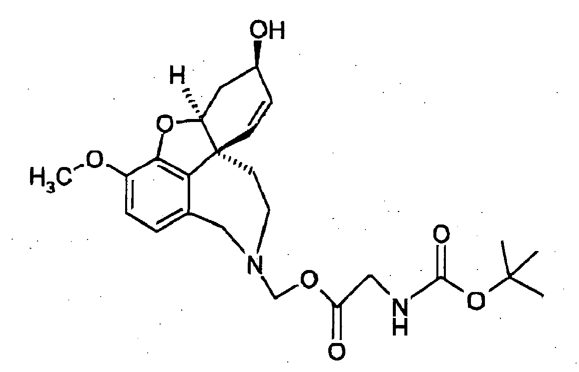

To a solution of N-Boc-glycine chloromethylester (1.0 mmol) and norgalanthamine (1.0 mmol) in dry DMF (2.0 mL) triethylamine (3 mmol) was added dropwise and the reaction stirred under nitrogen for 3 days. The triethylammonium chloride formed was filtered and washed with dry ether and the filtrate rotoevaporated to dryness. The residue was redissolved in dry acetone (2 ml) upon heating and left to stand overnight at 4°C for additional precipitation of the triethylammonium salt. After renewed filtration and rotoevaporation the mixture was chromatographed on silica using ethyl acetate / petrol ether. The target product was isolated as an oil.

¹³H NMR (DMSO-d₆)_δ 28.5, 33.9, 37.9, 42.0, 48.2, 51.2, 56.2, 56.9, 61.9, 79.5, 79.9, 88.8, 111.8, 121.3, 126.6, 129.8, 130.8, 133:6, 145.8, 148.2, 156.3, 169.6.

Example 3: 2-tert-Butoxycarbonylarnino-3-phenylpropionic acetic acid (N-norgalantha-minyl)-methyl ester

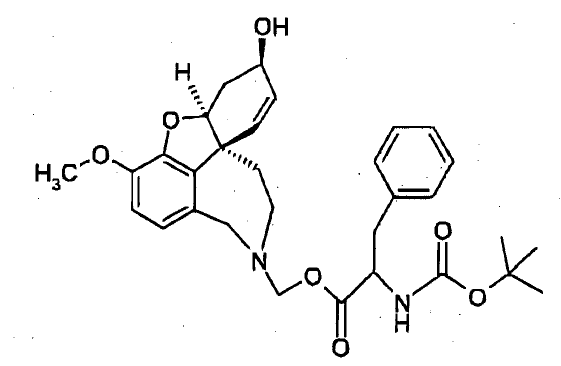

This compound was prepared using the procedure of example 2 with N-Boc-phenylalanine chloromethylester.

¹³H NMR (DMSO-d₆)_δ 28.5, 33.9, 36.9, 37.9, 48.2, 51.2, 54.6, 56.2, 56.9, 61.9, 79.5, 80.2, 88.8, 111.8, 121.3, 126.0, 126.6, 127.8, 128.7, 129.8, 130.8, 133.6, 139.5, 145.8, 148.2, 156.0, 171.6.

Example 4: (3R,4aS,9bS)-9-Dimethylaminomethyl-6-methoxy-3,4,4a,9b-tetrahydro-9b-vinyl-dibenzofuran-3-ol; (= 10,11-Seco-11,12-dehydro-10-methyl-galanthamine)

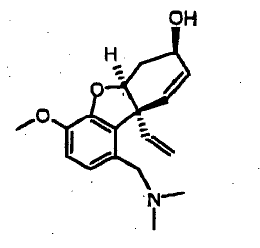

A solution of N-methylgalanthaminium iodide (5.0 g, 11.6 mmol) in 35% aqueous potassium hydroxide (150 mL) is heated under reflux for 48 hrs, diluted with water (200 mL) and acidified using conc. hydrochloric acid to pH = 3-4 and extracted with dichloromethane (2 x 50 mL) to remove non-basic compounds. The aqueous phase is basified using conc. ammonia to pH 12 and extracted using dichloromethane (4 x 100 mL). The combined organic extracts are washed with brine (2 x 50 mL), dried using sodium sulfate and rotoevaporated to obtain the crude product which is purified by MPLC (200 g SiO₂, chloroform: methanol = 99 : 1 + 1 % conc. ammonia). The product is obtained as yellow oil (2.5 g, 71 % d. Th.). The fumarate (colourless crystals) and oxalate salt (off-white crystals) where obtained in the usual way: m.p.: 151-153°C (fumarate), 116-118°C (oxalate). [α]_{D}²⁰= -56.5° (0.212 g/100 mL H₂O) (fumarate).

| fumarate: | oxalate |
|---|---|
| C₁₈H₂₅NO₃ * 1.0 C₄H₄O₄ | C₁₈H₂₅NO₃ * 1.0 C₂H₂O₄ * 0.75 H₂O |
| Calcd.: C, 62.99; H, 6.97; N, 3.34 | Calcd.: C, 59.32; H, 6.60; N, 3.46 |
| Found: C, 62.89; H, 6.62; N, 3.32 | Found.: C, 59.48; H, 6.31; N, 3.38 |

¹H-NMR (CDCl₃): δ 6.83 (d, *J*= 8.4 Hz, 1H), 6.72 (d, *J*= 8.4 Hz, 1H), 6.13-5.95 (m, 3H), 5.32 (dd, *J=* 10.3, 1.1 Hz, 1H), 5.25 (dd, *J=* 18.3, 1.1 Hz), 4.63 (b, 1H), 4.15 (b, 1H), 3.85 (s, 3H), 3.58 (d, *J=* 12.8 Hz, 1H), 3.07 (d, *J=* 12.8 Hz, 1H), 2.56 (m, 1H), 2.15 (s, 6H), 1.96 (ddd, *J*= 16.2, 4,9, 2.3 Hz, 1H);¹³C-NMR(CDCl₃):δ 146.6 (s), 144.1 (s), 139.0 (t), 132.2 (s), 128.6 (s), 128.1 (d), 127.8 (d), 123.6 (d), 117.3 (t), 111.1 (d), 86.0 (d), 62.0 (t), 59.7 (t), 55.7 (q), 52.9 (s), 44.7 (q), 28.6 (t)

Example 5: (3R,4aS,9bS)-6-Methoxy-9-methylaminomethyl-3,4,4a,9b-tetrahydro-9b-vinyl-dibenzofuran-3-ol; (= 10,11-Seco-11,12-dehydro-galanthamine)

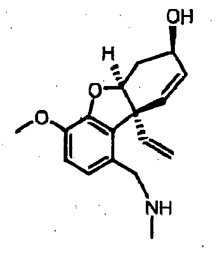

3-Chloroperbenzoic acid (0.38 g, 75%ig, 1.66 mmol) is added to a solution of (3R,4aS,9bS)-9-dimethylammomemyl-6-memoxy-3,4,4a,9b-tetrahydro-9b-vinyl-dibenzofuran-3-ol (0.50 g, 1.66 mmol) in dichloromethane (35 mL) and then stirred for 30 minutes at room temperature. After adding a solution of iron(II)sulfate-heptahydrate (0.23 g, 0.83 mmol) in methanol (5 mL) it is then stirred for another 20 minutes at room temperature. Then 2N hydrochloric acid (30 mL) is added, stirred for 5 minutes and most of the dichloromethane is removed by rotoevaporation. The remaining aqueous phase is washed with diethyl ether (4 x 20 mL), basified to pH 12 using concentrated ammonia and then extracted with dichloromethane (4 x 40 mL). The combined organic phases are washed with saturated sodium chloride solution (30 mL), dried using sodium sulphate, filtered and the solvent is again removed by rotoevaporation to obtain the crude product which is then further purified using MPLC (Büchi, 110 g SiO₂, chloroform: methanol 97 : 3 + 1 % concentrated ammonia) and obtained as a yellow oil (0.30 g, 63 % d. Th.). The oxalate is prepared in the usual way and obtained as colourless crystals, 0.37 g, 59 % d. Th., m.p. 127-129°. The purity is checked by TLC (chloroform: methanol = 9:1+1% conc. ammonia, Rf= 0.35). [α]_{D}²⁰= -41.8° (0.220 g/100 mL H₂O) (Oxalate)
C₁₇H₂₁NO₃ * 1.0 C₂H₂O₄ * 0.5 H₂O
Calcd.: C, 59.06; H, 6.26; N, 3.62
Found: C, 59.35; H, 6.00; N, 3.56

¹H-NMR (CDCl₃): δ 6.88 (d, *J*= 8.4 Hz, 1H), 6.75 (d, *J*= 8.4 Hz, 1H), 6.15-5.82 (m, 3H), 4.67 (b, 1H), 4.09-4.20 (m, 1H), 3.85 (s, 3H), 3.68 (s, 2H), 2.52-2.49 (m, 1H), 2.42 (s, 3H), 1.97 (ddd, *J*= 16.2,4.9,2.3 Hz, 1H); ¹³C-NMR (CDCl₃): δ 146.6 (s), 144.0 (s), 139.4 (d), 131.6 (s), 129.5 (s), 128.9 (d), 127.2 (d), 122.7 (d), 117.6 (t), 111.8 (d), 86.0 (d), 62.0 (d), 55.9 (q), 52.8 (t), 51.1 (s), 36.0 (q), 28.8 (t)

Example 6: (3R,4aS,9bS)-9-Dimethylaminomethyl-9b-ethyl-6-methoxy-3,4,4a,9b-tetrahydro-dibenzofuran-3-ol; (= 10,11-Seco-10-methyl-galanthamine)

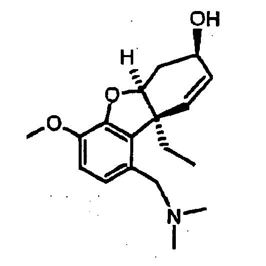

Palladium (10%) on active carbon (90 mg) is pre-hydrogenated in methanol (40 mL) and conc. acetic acid (2 mL) in the Parr-apparatus at 10 psi and room temperature for 45 minutes. After adding (3R,4aS,9bS)-9-dimethylaminomethyl-6-methoxy-3,4,4a,9b-tetralhydro-9b-vinyl-dibenzofuran-3-ol (0.90 g, 2.99 mmol) it is then hydrated for 8 hrs. at 15-20 psi and room temperature. The catalyst is then filtered and the solvent is removed by rotoevaporation. The residue is then dissolved in water (100 mL), basified using cone. ammonia and extracted using dichloromethane (5 x 40 mL). The combined aqueous phases are washed with a saturated sodium chloride solution (2 x 20 mL), dried using sodium sulphate and the solvent is removed by rotoevaporation. It is then further purified using MPLC (Büchi, 110 g SiO₂, chloroform : methanol = 98 : 2 + 1 % conc. ammonia), obtained as a colourless oil (0.80 g, 88 %) and converted to the hydrochloride m.p. 248-249°. [α]_{D}²⁰= -47.3° (0.220 g/100 mL H₂O). TLC chloroform : methanol = 9 : 1 + 1 % cone. ammonia, Rf = 0.45.
C₁₈H₂₅NO₃ * 2.0 HCl
Calcd.: C, 57.45; H, 7.23; N, 3.72
Found: C, 57.95; H, 6.85; N, 3.48

¹H-NMR (CDCl₃):_δ 6.78 (d, *J* = 8.3 Hz, 1H), 6.67 (d, *J* = 8.3 Hz, 1 H), 6.12 (d, *J* = 10.2 Hz, 1H), 5.89 (dd, *J =* 10.2, 4.3 Hz, 1H), 4.74 (b, 1H), 4.19-4.09 (m, 1H), 3.84 (s, 3H), 3.54 (d, J= 12.9 Hz, 1H), 3.19 (d, *J =* 12.9 Hz, 1H), 2.53-2.32 (m, 1H), 1.94-2.13 (m, 2H), 1.69 (ddd, *J*= 16.2, 4.9, 2.3 Hz, 1H), 0.85 (t, *J* = 7.6 Hz, 3H); ¹³C-NMR (CDCl₃): δ 146.8 (s), 144.4 (s), 131.6 (d), 128.2 (s), 127.7 (d), 123.6 (d), 110.6 (d), 83.8 (d), 62.7 (d), 61.6 (s), 55.7 (q), 51.1 (s), 45.2 (q), 31.6 (t), 27.5 (t),

Example 7: 3.3.4. (3R,4aS,9bS)-9b-Ethyl-9-methylaminomethyl-6-methoxy-3,4,4a,9b-tetrahydro-dibenzofuran-3-ol; (= 10,11-Seco-galanthamine)

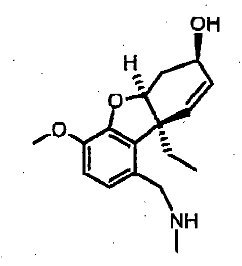

Following the procedure of example 6 using (3R,4aS,9bS)-9-Dimethylaminomethyl-9b-ethyl-6-methoxy-3,4,4a,9b-tetrahydro-dibenzofuran-3-ol the pure product is obtained as a yellow oil (0.17 g, 59 % d. Th.) and converted to the oxalate and fumarate.
M.p. (oxalate) 162-164°, [α]_{D}²⁰= -51.2° (0.146 g/100 mL H₂O) (oxalate).
TLC chloroform : Methanol = 9:1+1% conc. ammonia, Rf = 0.39

| fumarate: | oxalate |
|---|---|
| C₁₇H₂₃NO₃ * 1 C₄H₄O₄ * 0.33 H₂O | C₁₇H₂₃NO₃ * 1 C₂H₂O₄ * 0.25 H₂O |
| Calcd.: C, 61.31; H, 6.78; N, 3,40 | Calcd.: C, 59.44; H, 6.69; N, 3.65 |
| Found: C, 61.22; H, 6.67; N, 3.22 | Found; C, 59.53; H, 6.78; N, 3.65 |

¹H-NMR (CDCl₃): δ 6.85 (d, *J*= 8.4 Hz, 1H), 6.73 (d, *J=* 8.4 Hz, 1H), 5.97-5.92 (m, 2H), 4.74 (dd, *J=* 5.8, 3.5 Hz, 1H), 4.22-4.12 (m, 1H), 3.84 (s, 3H), 3.74 (d, *J* = 7.2 Hz, 2H), 2.48 (s, 3H), 2.45-2.28 (m, 2H), 2.20-1.62 (m, 5H), 0.85 (t, J = 7.46, 3H); ¹³C-NMR (CDCl₃): δ 146.6 (s), 144.2 (s), 131.1 (s), 131.0 (d), 128.9 (s), 128.8 (d), 122.3 (d), 111.1 (d), 83.8 (d), 62.8 (d), 55.8 (q), 51.0 (s), 36.3 (q), 32.5 (t), 29.1 (t),

Example 8: (4aS,6R,8aS)-4a,3,9,10,11,12-Hexahydro-3-methoxy-11-methyl-6H-benzofuro[3a,3,2-ef][2]benzazepin-5-yl-β-D-glucopyranosiduronic acid (= Galanthamine-3-glucuronide)

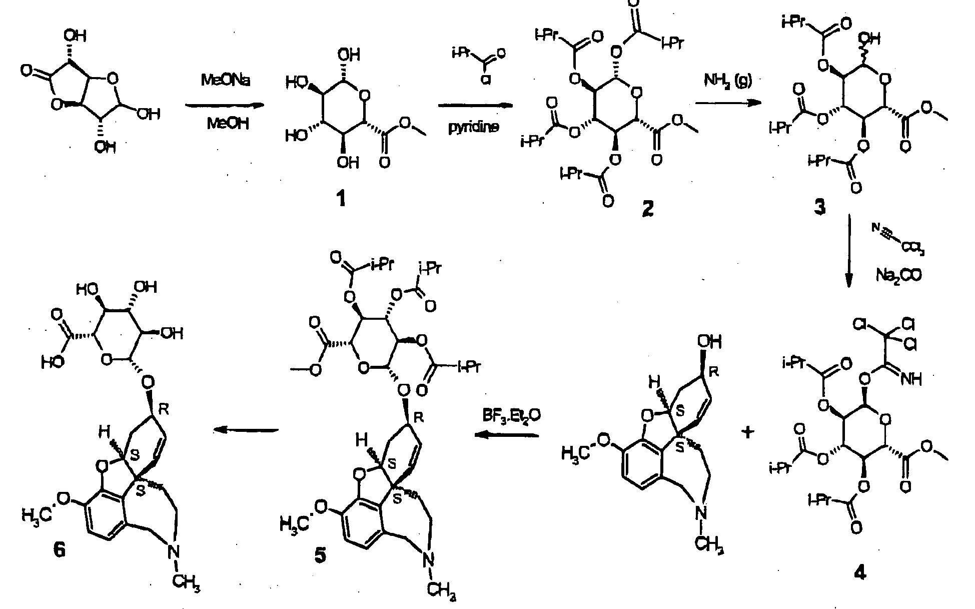

Step 1: Methyl 1,2,3,4-tetra-*O*-isobutyril-ß-D-glucopyranuronate (2)

To a solution of NaOMe (26 mg, 0.48 mmol) in MeOH (150 mL) was added glucurono-6,3-lactone (20.6 g, 154 mmol) in portions with stirring until dissolved. The solvent was then removed in vacuo, the residue taken up in pyridine (85 mL, 1.08 mol) and the solution cooled to 0°C. Isobutyryl chloride (110 mL, 1.06 mol) in CH₂Cl₂ (70 mL) was then added with strong mechanical stirring at a rate that kept the temperature below 10°C, and the reaction mixture was left at room temperature overnight. More CH₂Cl₂ (100 mL) was then added and the solution washed with water (400 mL), 2 M HCl (3×50 mL), saturated sodium bicarbonate (5×50 mL) and brine (50 mL). After drying, filtering and evaporating in vacuo, a gum was obtained which crystallized on trituration with petroleum ether (40-60°C). Filtration and drying at 40°C in a vacuum oven yielded the title product. Recrystallisation from MeOH or petrol ether afforded the pure β isomer 2 as needles, mp 127°C, (21.6 g, 37%, from mother liquid some more product could be isolated) [α]_{D} = +11.12 (c 1.7 CHC13); δ_{H} (300 MHz, CDCl3): 5.78 (d, *J=*8 Hz), 5.39 (t, *J*=9.5 *Hz),* 5.25 (t, *J=9.5* Hz), 5.23 (dd, *J=*9.5, 8 Hz), 4.19 (d, *J*=9.5 Hz),3.75 (s, OMe), 2.65-2.45 (m, 4xC*H*Me₂), 1.17-1.07 (m, 4xCH*Me₂*),

An alternative procedure with pivaloyl chloride was also used to prepare methyl 1,2,3,4-tetra-*O*-pivaloyl-β-D-glucopyranuronate in 21% (the isolation and crystallization of compound 2 was easier).

### Step 2: Methyl 2,3,4-tri-O-isobutyryl-D-glucopyranuronate (3)

Ammonia gas pre-dried by passing it through a bed of sodium hydroxide was bubbled through CH₂Cl₂ (200mL) at -4°C over 1 h at a rate which kept the temperature below 0°C. The above methyl 1,2,3,4-tetra-O-isobutyryl-β-D-glucopyranuronate (3.0 g, 8 mmol) was added and the solution stirred at 0°C for 3h and then left at room temperature for 20h. Nitrogen gas was bubbled through the solution for 30 min. and it was extracted with ice-cold 10% aqueous HCl, then water. The organic phase was dried over Na₂SO₄ filtered and solvent removed in vacuo to leave the crude product. Recrystallization from CHCl₃:PE afforded the pure microcrystalline α-epimer, mp 89°C. δH (300 MHz, CDC13): 5.65 (t, *J* =10 Hz), 5.54 (d, *J=3.5* Hz), 4.92 (dd, *J*=10,3.5 Hz), 4.60 (d, *J*=10 Hz), 3.75 (s, OMe), 2.61-2.43 (m, 4×C*H*Me₂), 1.20-1.05 (m, 4×C*H*Me₂).

### Step 3: Methyl 2,3,4-tri-O-isobutyril-1-O-trichloroacetimidoyl-□-D-glucopyranuronate (4)

To a stirred solution of methyl 2,3,4-tri-*O*-isobutyryl-D-glucopyranuronate 3 (418 g, 1 mmol) in CH₂Cl₂ (5 mL) was added trichloroacetonitrile (0.4 mL, 3.7 mmol), followed by anhydrous potassium carbonate (83 mg, 0.6 mmol), and the mixture stirred for 40h. It was filtered through a short pad of silica and eluted with ether. Filtration and evaporation in vacuo then yielded the title product 4 as a semi crystalline gum which crystallized from dry isopropanol as white prisms, mp 108°C (422 mg, 75%). δH (300 MHz, CDCl3): 8.72 (s, NH), 6.66 (d, *J*=3.5 Hz), 5.70 (t, J=10 Hz), 5.30 70 (t, *J*=10 Hz), 5.20 (dd, *J*=10,3.5 Hz), 4.51 (d, *J=*10 Hz), 3.75 (s, OMe), 2.60-2.43 (m, 3×C*H*Me₂), 1.17-1.06 (m, 3×CH*Me*₂).

### Step 4: Galanthamine-6-methyl 2,3,4-tri-O-isobutyryl-β-D-glucopyranuronate (5)

A suspension of dried galanthamine hydrobromide (92 mg, 0.25 mmol) and the above methyl 2,3,4-tri-O-isobutyryl-1-O-trichloroacetimidoyl-β-D-glucopyranuronate 4 (282 mg, 0.5 mmol) in dry CH₂Cl₂ (10 mL) containing 4□ molecular sieves was stirred under argon at room temperature, while BF₃·Et₂O (0.1 mL, 0.5 mmol) was added. After 1h, virtually all of the starting materials had dissolved and stirring was continued for 2 days. More CH₂Cl₂ (20 mL) was added, the solution washed with saturated aq. sodium bicarbonate (10 mL), water and brine before being dried. Filtration and evaporation in vacuo afforded a semisolid residue, which was purified with MPLC on silica. Elution with CHCl₃/MeOH 97:3-20 gave 75 mg of the glucuronide. Trituration with EtOH yielded 30 mg of pure 5.

### Step 5: (4aS,6R,8aS)-4a,5,9,10,11,12-Hexahydro-3-methoxy-11-methyl-6H-benzofuro[3a,3,2-ef][2]benzazepin-6-yl-β-D-glucopyranosiduronic acid (Galanthamine-3-glucuronide) (6)

2M-NaOH (2.0 mL) was added to a stirred suspension of the glucuronate 5 (30 mg) in MeOH (4 mL), and the mixture left overnight. The solution was then acidified with glacial acetic acid to pH 5.5, the solvent evaporated and purified over silica with CHCl₃: MeOH (saturated with dry NH₃) 95:5. The product- fraction was freeze-dried to afford 14 mg of 6 as a white powder, m.p. 238° (dec.).

¹H NMR (MeOD, 200 MHz): 1.63-1.73 (m, 1H), 2.02-2.21 (m, 2H), 2.38 (s, 3H), 2,43-2.53 (m, 1H), 2.99-3.06 (m, 1H), 3.19-3.33 (m, 1H), 3.47-3.49 (m, 1H), 3.65-3.71 (d, 1H, J= 14.9 Hz), 3.78 (s, 3H), 4.05-4.13 (d, 1H, J= 14.9 Hz), 4.58 (m, 1H), 5.85-5.94 (dd, 1H, J₂=4.8 Hz, J₂ = 10.2 Hz), 6.15-6.21 (d, 1H, J₂= 10.2 Hz), 6.63-6.77 (m, 2H)

¹³C NMR (MeOD, 200 MHz): 23.22, 28.65, 34.57, 42.02, 43.33, 48.09, 54.03, 55.64, 60.43, 88.54, 112.18, 122.30, 127.16, 127.70, 128.64, 133.49, 144.65, 146.39

### Example 9: Galauthamine-3,64-di-β-D-glucuronide

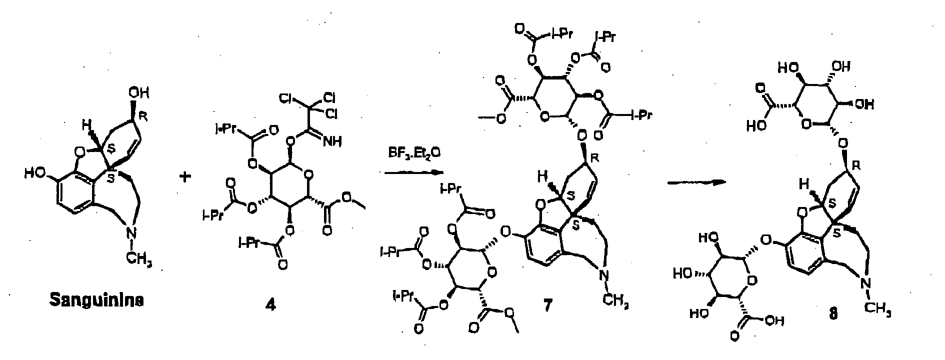

### Step 1: Galanthamine-3,6-di(methyl 2,3,4-tri-O-isobutyryl-β-D-glucopyranuronate) (7)

Following the procedure for the preparation of Galanthamine-6-methyl 2,3,4-tri-O-isobutyryl-β-D-glucopyranuronate but using sanguinine (137 mg, 0.5 mmol) and the above imidate 4 (1.12 g, 2 mmol) in dry CH₂Cl₂ (10 mL) afforded, after analogous workup a semisolid residue, that was purified with MPLC on silica. Elution with CHCl₃/MeOH 97:3-20 gave the crude product (180 mg). Trituration with EtOH yielded 130 mg of the pure product 7.

### Step 2: Galanthamine-3.6-β-D-diglucuronide (8)

2M-NaOH (2.0 mL) was added to a stirred suspension of the above glucuronate 7 (130 mg) in MeOH (4 mL), and the mixture left overnight. The solution was then acidified with glacial acetic acid to pH 5.5, the solvents removed by freeze drying and the product chromatographed on silica using CHCl₃: MeOH (saturated with dry NH₃) 95:5. gave 48 mg (63.5%) of the product 8.

¹H NMR (CDCl₃, 200 MHz): 1.60-1.72 (m, 2H), 1.82-2,6 (m, 10 H), 2.88-3.30 (m, 3H), 3.50-3.67 (m, 6H), 3.80-4.20 (m, 3H), 4.30-4.70 (m, 1H), 4.94-5.30 (m, 6H), 5.76-6.21 (m, 2H), 6.42-6.56 (m, 1H), 6.74-6.86 (m, 1H)

### Example 10: 3-Nicotinoyl-galanthamine

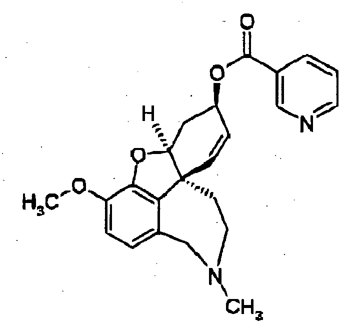

A solution of galanthamine (431 mg, 1.5 mmol) in dry pyridine (25 mL) was treated with nicotinoyl chloride (240 mg, 1.7 mmol) and 4-N,N-dimethylaminepyridine (5 mg) at 0° and the solution stirred to room temp. for 2 hrs. followed by heating to 45° for 1 hr. The reaction mixture was poured on water (150 mL) and the pH adjusted to 8.0 followed by extraction with dichloromethane. The organic extract was washed with water and brine, dried (sodium sulphate) and evaporated to give the crude product (480 mg, 81.5%) ¹³H NMR (DMSO-d₆)₋δ 27.7, 34.3, 41.7, 47.8, 53.6, 55.9, 60.3, 63.2, 86.2, 111.5, 121.3, 122.1, 122.7, 126.0, 129.2, 130.6, 131.9, 136.4, 143.9, 146.5, 150.4, 151.5, 166.0.

This product was converted to the dihydrobromide salt by dissolution in a minimum amount of warm 40% hydrobromic acid followed by cooling and obtained as colorless crystals.

Anal. calcd. for C₂₃H₂₄N₂O₄, 2 HBr. 0.33 H₂O C 49.31; H 4.80; N 5.00. Found C 49.10; H 5.05; N 4.85.

### Example 11: (+-)-8-fluorogalanthamine:

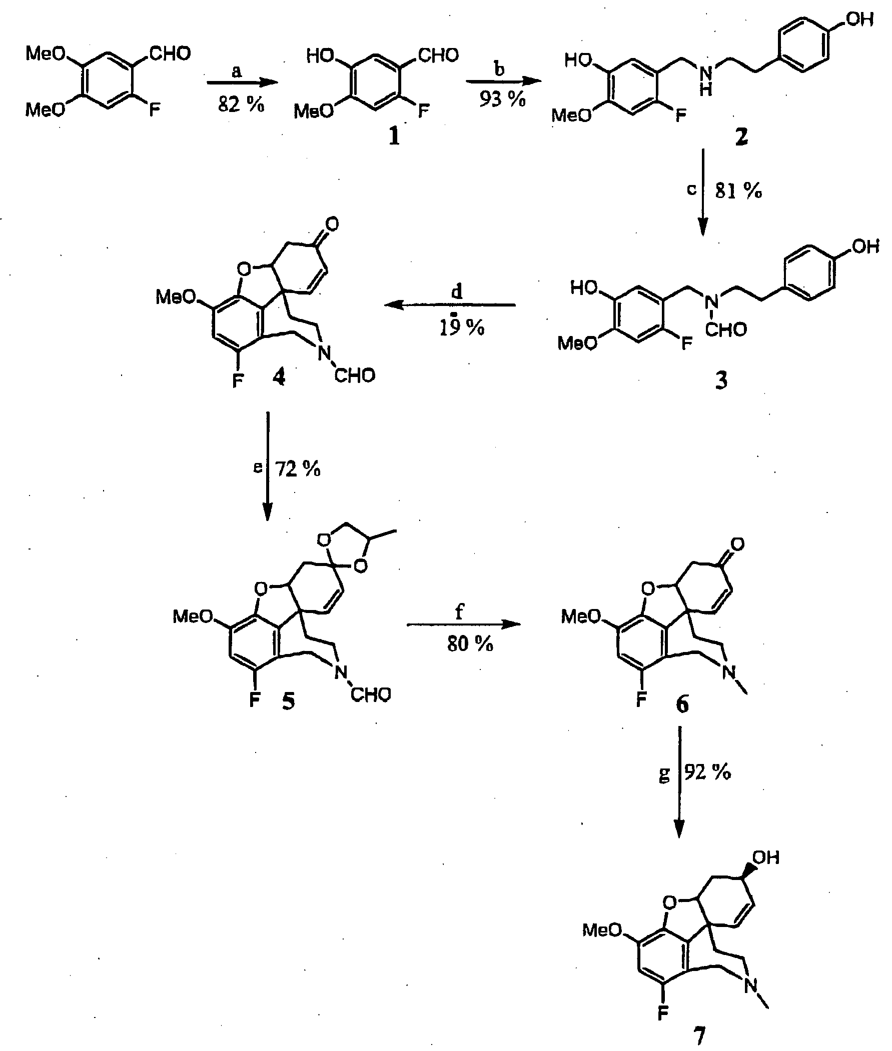

a) H₂SO₄, 90 °C b)1. 4-hydrvxy-phenylethyl amine, toluol/n-buthanol, reflux 2. methanol, NaBH₄ c) ethylformiat, formic acide, DMF, dioxan, reflux d) K₃[Fe(CN)₆], K₂CO₃, toluol/water, 50 °C e)1,2-propanediol, PTSA, toluene, reflux f) LiAIH₄, THF, 2 N HCl g) L-selectride, THF

### Step 1:2-Fluoro-5-hydroxy-4-methoxy benzaldehyde (1):

Sulphuric acid (50 ml, 95-98 %) was heated with stirring to the 90-95 °C under a dry nitrogen and 4,5-dimethoxy-2-fluoro benzaldehyde (10:1 g, 54.8 mmol) added quickly and this mixture was stirred at the same temperature for 3.5 h. Reaction was followed by HPLC and found to be complete after this time. The reaction mixture was poured on crushed ice (150 g) and the white slurry obtained was heated to 65 °C and allowed to cool in the fridge overnight. The white precipitate was filtered and washed with water (2x100 ml). The wet cake was dried in the desiccator under reduced pressure to afford the product (7.6 g, 82 %, HPLC 95 %, m.p.: 146-148) as off white crystals.

### Step 2: 4-Fluoro-5-{[2-(4-hydroxyphenyl)ethylamino]-methyl]-2-methoxy-phenol (2):

A solution of 1 (7.6 g, 45 mmol) and tyramine (6.7 g, 49 mmol) in dry toluene (250 ml) and n-butanol (250 ml) was heated and stirred to reflux for 5 h on the Dean-Stark apparatus to remove the water. Reaction development was controlled by TLC (MeOH:CH₂Cl₂ 1:9) and reaction was found to be complete after this time. Solvents were rotoevaporated and residue was dissolved in dry methanol (500 ml). NaBH₄ (1.8 g, 45 mmol) was added at the temperature 0-5 °C and this mixture was stirred overnight while the temperature was raised to room temperature and a white solid precipitated from the reaction mixture. The solid was filtered and washed with cold methanol (2x50 ml). The white, wet cake was dried in the desiccator at reduced pressure to give the product (9.6 g, 74 %, HPLC> 99 %) as a white powder. The filtrate was rotaevaporated to give a brown slurry (3.6 g), which was chromatographed on silica (dichloromathane/methanol, gradient 0-10 %) to give another (2.5 g, 19 %, HPLC > 99 %) of product as a off white powder (total yield 93%, m.p.: 160-162 °C).
*¹H NMR (MeOD, 200 MHz):* 2.69 (s, broad, 4H), 3.66 (s, 2H), 3.80 (s, 3H), 6.66-6.77 (m, 4H), 6.96-7.00 (m, 2H).

### Step 3: N-[(2-fluoro-5-hydroxy-4-methoxyphenyl)methyl]-N-[2-(4-hydroxyphenyl)ethyl]-formamide (3):

To a suspension of 2 (7.63 g, 26.1 mmol) in dioxane (50 ml) a solution of ethyl formiate (3.1 ml, 37.7 mmol), DMF (1.5 ml) and formic acid (0.25 ml, 6.62 mmol) was added dropwise and the reaction mixture was heated under argon to reflux for 10 h. The reaction development was controlled by HPLC and showed complete conversion after this time. Volatiles were removed under reduced pressure, the residue was dissolved in methanol (32 ml) and poured on crushed ice (160 ml), the white precipitate formed was stirred magnetically for 1 h, filtered, washed with water (3x100 ml) and dried to weight to afford the product (6.8 g, 81.3 %, HPLC > 99 %, m.p.: 153-168 °C) as a white powder.
*¹H NMR (DMSO, 200 MHz):* 2.49-2.67 (m, 2H), 3.15-3.29 (m, 2H), 3.75 (s, 3H), 4.28-4.35 (d, 2H, J₂= 13.89 Hz), 6.64-6.95 (m, 6H), 7.84 (s, 0.5 H), 8.20 (s, 0.5 H), 8.95-9.00 (d, 1H, 10.17 Hz), 9.18-9.20 (d, 1H, J= 2.44 Hz).

### Step 4: 4α,5,9,10,11,12-Hexahydro-1-fluoro-3-methoxy-11-formyl-6H-benzofuro[3a,3,2-ef] benzazepine-6-one (4):

To the vigorously stirred biphasic mixture of potassium carbonate (13.2 g, 95,5 mmol) and potassium hexacyanoferrate (28 g, 85,4 mmol) in toluene (580 ml) and water (120 ml), preheated to 50 °C, finely pulverized 3 (6.83 g, 21.4 mmol) was added in one portion and this suspension was heated at 50-60 °C with intense stirring for 1 h. After this time the reaction mixture was filtered trough the pad of celite, the toluene phase separated and the water phase was extracted with toluene (2x100 ml). The combined organic phases were dried (Na₂SO₄) and roto-evaporated under reduced pressure to afford the product (1.3 g, 19 %, HPLC 98 %) as a white powder.
*¹H NMR* (*DMSO, 200 MHz):* 1.75-1.93 (m, 1H), 2.15-2.30 (m, 1H), 2.73-2.83 (m, 1H), 3.00-3.12 (m, 1H), 3.40 (s, 4H), 3.98-4.13 (m, 1H), 4.28-4.35 (m, 0.5 H), 4.51-4.97 (m, 2H), 5.27-5.34 (d, 0.5 H, J= 15,45 Hz), 5.94-6.00 (d, 1H, J= 10.37 Hz), 6.77-6.86 (m, 1H), 7.15-7.26 (m, 1H), 8.10-8.15 (d, 1H, J= 8.99 Hz)
*¹³C NMR* (*DMSO, 200 MHz):* 34.02, 37.21, 37.32, 45.45, 49.33, 49.53, 56.05, 87.29, 100.20, 100.34, 100.77, 100.90, 114.55, 114.93, 115.08, 126.66, 130.83, 130.93, 143.12, 143.43, 143.64, 143.76, 144.29; 144.52, 162.39, 162.62, 194.77.

### Step 5: 1-Bromo-4a,5,9,10-tetrahydro-3-methoxy-spiro[6H-benzofuro[3a,3,2-ef][2]benzazepine-6,2'-[1,3]dioxolane]-11(12H)-carboxaldehyde (5):

To the solution of 4 (1.084 g, 3.42 mmol) in toluene (10 ml) a solution of 4-toluene sulphonic acid (0.02 g, 0.116 mmol) in 1,2-propane-diol (1.13 ml) was added and the mixture heated to the reflux for 1 h while the water was removed using a Dean-Stark apparatus. Another portion of 4-toluene sulphonic acid (0.05 g) in 1,2-propanediol (0.65 ml) was added and heating continued for another 5 h. Reaction development was controlled by HPLC and the reaction found to be complete after this time. The reaction mixture was cooled to room temperature and extracted with acetic acid (2x25 ml, 10 % in water), sodium hydrogen carbonate (2x25 ml, 10% in water) and brine (1x25 ml). The toluene solution was dried (Na₂SO₄) and evaporated to give a crude product (1.32 g) as an amber oil. This was crystallized using i-propanol and ligroin to give product (0.92 g, 72 %), as a colourless crystals.
*¹H NMR (CDCl₃, 200 MHz):* 0.74-2.66 (m, 10 H), 2.98-4.86 (m, 8 H), 5.44-5.74 (m, 1H), 6.34-6.39 (m, 1H), 7.98-8.03 (m, 1H).

### (+-)-8- Fluoro-Narwedin (6);

To the solution of 5 (0.91 g, 2.43 mmol) in dry THF (15 ml) lithium aluminium hydride (1.21 ml, 2.3 mol suspension in THF) was added at 0-5 °C under a continuous stream of dry nitrogen and this mixture was stirred for 1 h.. Another portion of lithium aluminium hydride (0.605 ml, 2.3 mmol suspension in THF) was added and stirring continued for additional 1 h while the temperature raised slowly to room temperature. Reaction development was controlled by HPLC and no starting material was detected after this time. The reaction mixture was quenched with water/THF 1:1 (20 ml) and volatiles removed under reduced pressure. The residue was dissolved in 2N-hydrochloric acid (25 ml) and stirred at room temperature for 30 min. The clear solution was than treated with ammonia to pH 12 and extracted with ethyl acetate (3x50 ml). The combined organic phases were dried (Na₂SO₄), treated with charcoal, filtered and evaporated to dryness to give 720 mg of the crude product as an brown oil. Chromatography on silica using 7 N NH₃ in MeOH : CH₂Cl₂ 5 : 95 as solvents afforded the product (590 mg, yield 80 %, HPLC 97 %) as an amber oil.
*¹H NMR (CDCl₃, 200 MHz):* 1.77-1.84 (m, 1H), 2.09-2.24 (m, 1H), 2.38 (s, 3H), 2.60-2.71 (m, 1H), 2.98-3.11 (m, 3H), 3.64-3.72 (m, 4H), 4.03-4.11 (d, 1H, J= 15.65 Hz), 4.65 (s, 1H), 5.93-5.98 (d, 1H, J=10.56 Hz), 6.40-6.46 (d, 1H, J=11.34 Hz), 6.84-6.89 (m, 1H) *¹³C NMR (CDCl₃*, *200 MHz):* 33.41, 37.22, 43.18, 49.42, 49.46, 51.91, 51.99, 54,13, 56.20, 88.12, 99.99, 100.58, 114.98, 115.34, 127.31, 131.33, 131.43, 142.84, 143.51, 143.71, 144.11, 152.42, 157.18; 194.13.

### (+-)-8-fluorogalanthamine (7):

To the solution of 6 (500 mg, 1.64 mmol) in dry THF (30 ml) L-Selectride (1.50 ml, 1 M solution in THF) was added dropwise at -5 to 0 °C under dry nitrogen and this mixture was stirred at the same temperature for 30 min. The reaction was monitored by HPLC and no starting material was detected after this time. The reaction was quenched using water/THF 2:1 (50 ml) and solvents were removed under reduced pressure. The residue was dissolved in 2N-hydrochloric acid (100 ml) and kept overnight in the fridge. The aqueous solution was than washed with diethyl ether (2x30 ml) and ammonia was added to pH 12. The aqueous phase was extracted using ethyl acetate (3x100 ml), the combined organic phases were washed with brine (50 ml), dried (Na₂SO₄) and evaporated to afford the crude product (515 mg) as a clear, slightly yellow oil which was purified by chromatography on silica using MeOH:CH₂Cl₂ 9:1 to afford the product (0.46 g, 92 %, HPLC> 99 %) as a white powder.
*¹H NMR (CDCl₃, 400 MHz):* 1.25 (s, 1H), 1.55-1.67 (m, 1 H), 1.92-2.10 (m, 2 H), 2.41 (s, 4H), 2.62-2.70 (m, 1H), 2.98-3.29 (m, 2H), 3.72-3.78 (d, 1H), 3.81 (s, 3H), 4.07-4.20 (m, 2H), 4.60 (s,1H), 6.03 (s, 2H), 6.47-6.49 (d, 1H),
*¹³C NMR (CDCl₃, 400 MHz):* 30.11 (C-5), 34.31 (C-9), 43.10 (N-CH₃), 49.21 (C-8a), 52.15 (C-10), 54.32 (OCH₃), 56.55 (C-12), 62.37 (C-6), 89.29 (C-4a), 99.86 (C-2), 100.16 (C-12a), 126.89 (C-12b), 134.25 (C-8), 134.30 (C-7), 142.09 (C-3a), 144.23 (C-3), 154.31 (C-1), 156.69 (C-1).

## Claims

1. Method for improvement of blood-brain barrier permeability and/or brain-to-plasma distribution ratio of a cholinergic enhancer molecule, whether a cholinergic agonist or APL and/or a cholinesterase inhibitor and/or a neuroprotective agent, by modification of at least one of the residues R1, R2, R3, R4 and/or R5 of the base structure(s) of formula (III) so as to enhance transport into the brain and compound concentration therein 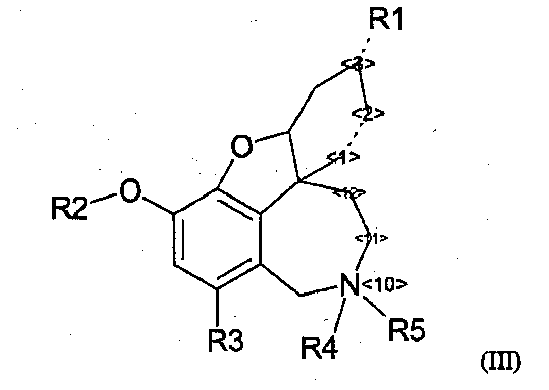 wherein the bond between positions <1> and <2> denotes a single- or double bond and the bonds <1> to <2> and <11> to <12> can be either a single or a double bond, and the bond between <10> and <11> is either a single bond or no bond, wherein the modified residues R1 - R5 are defined as follows:
R1:
a) if bond <3> to R1 is a double bond, then
R1 = O, NH, NOH, NOR6, N-CO-NH₂, N-CS-NH₂, N-C(=NH)-NH₂, N-NH-phenyl, N-NHR6, N-N(R6)₂, N-N=(CH₂)ₙ
with R6 = C₁-C₅ unbranched or branched, saturated or unsaturated (ar)alkyl, phenyl or benzyl and n=2-8
b) if bond <3> to R1 is a single bond, then
R1 = OH, SH, NH₂, NHR6, N(R6)₂, OR7, O-CR8R9-O-CO-CHR10-NR11R12
with R7 = C₁-C₂₂ unbranched or branched, (poly-)unsaturated or saturated alkyl, optionally containing an additional (ar)alkoxy or di(ar)alkylamino group, a sugar or sugar derivative residue, preferably glucuronic acid residue, or COR13,
where
R13 = R6 or R7 or pyridyl or dihydropyridyl or OR6, preferably methyl, 3-pyridyl, 4-pyridyl, 3-dihydropyridyl, 4-dihydropyridyl R8 and R9 are the same or different and any of H, Me, Ph or they together form a spiro-ring -(CH₂)ₙ- with n=4-6
R10 = H or the side chain of a natural amino acid including
R10,R11 together are forming a proline or hydroxy-proline derivative
R11 either is together with R10 forming a proline or hydroxy-proline derivative or is H
R12 is a carbamate protecting group including t-butoxycarbonyl, benzyloxycarbonyl and other N-protecting groups
R2: H, R7, or O-CR8R9-O-CO-CHR10-NR11R12
with the same definitions of R7-R12 as above
R3: H,F,Cl,Br,I, NH₂, NO₂,CN,CH₃
R4: H or CH₃
R5: If R4 = H, then R5 is an electron pair
if R4 = CH₃ then R5 is either hydrogen or a C₁-C₅ (ar)alkyl group, CH₂-O-CH₃, CH₂-O-CO-R6, CH₂-O-CR8R9-O-CO-CHR10-NR11R12 with the same definitions of R6 and R8-R12 as above, whereby in all the latter cases the nitrogen has an additional positive charge as well as a counterion, selected from chloride, bromide, iodide, sulphate, nitrate, hydrogensulfate, phosphate, methanesulphonate, tosylate or any other pharmaceutically acceptable anion
with the proviso that the resulting compound is not Galanthamine, Norgalanthamine, Sanguinine, Norsanguinine, Lycoramine, Norlycoramine, Lycoraminone, Narwedine, Nomarwedine,3-Amino-3-deoxy-galanthamine or 3-amino-3-deoxy-1,2-dihydro-galanthamine .

2. Method according to claim 1 wherein the bond <1> to >2> is a double bond and the bond between <3> and R1 is a single bond and bond <10> to <11> is a single or no bond and residues are
R1 = OH, OCO-(3-pyridyl)(=nicotinic acid residue), OCO-(3-methyl-3-pyridyl), OCO-(C₁-C₆ alkyl), OCO-(C₁-C₂₁ alkenyl), OCO-NH-(C₁-C₆ alkyl), OCO-(CH₂)ₓ-NH-COO-(C₁-C₆ alkyl), O-CH₂-O-(C₁-C₆ alkyl), O-(CH₂)ₓ-OCO-(C₁-C₆ alkyl), O-(CH₂)ₓ-OCO-(CH₂)ₓ-N-COO-(C₁-C₆ alkyl), O-(CH₂)ₓ-OCO-(CH₂)_{y}-aryl, OCOO-(C₁-C₆ aminalkyl), OCOO-(CH₂)ₓ-tetrahydrofuranyl, or a sugar, preferably glucuronic acid residue, wherein x=1, 2, 3 or 4 and y=0,1, 2, 3 or 4
R2 = H, CH₃, CO-(C₁-C₆ alkyl), CH₂-OCO-(CH₂)ₓ-aryl, or a sugar, preferably glucuronic acid residue
R3 = H, F or Br
R4 = H, C₁-C₆ alkyl, preferably CH₃, CO-(C₁-C₆ alkyl), CO-(3-pyridyl)(=nicotinic acid residue), CO-(3-methyl-3-pyridyl), CO-(CH-mercaptoalkyl)-(CH₂)ₓ-aryl, (CH₂)ₓ-OCO-(CH₂)ₓ-N-COO-(C₁-C₆ alkyl), (CH₂)ₓ-OCO-(CH-arylalkyl)-N-COO-(C₁-C₆ alkyl), wherein x=1, 2, 3 or 4
R5 = an electron pair or (CH₂)ₓ-O-(C₁-C₆ alkyl), (CH₂)ₓ-OCO-(C₁-C₆ alkyl), (CH₂)ₓ-OCO-(CH₂)ₓ-aryl, (CH₂)ₓ-OCO-(CH₂)ₓ-N-COO-(C₁-C₆ alkyl), wherein x=1, 2, 3 or 4 ; in case that bond <10> to <11> is a single bond the nitrogen has a positive charge and the counterion is chloride.

3. Use of compounds as defined in claim 1 for the preparation of a pro-drug or medicament with improved blood-brain barrier permeability compared to Galanthamine.

4. Derivatives of a base structure of the formula (III) 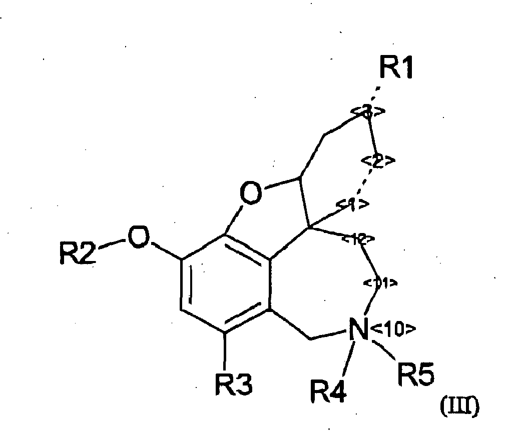 wherein the bond <1> to >2> is a double bond and the bond between <3> and R1 is a single bond and bond <10> to <11> is a single or no bond and residues are
R1 = OH, OCO-(3-pyridyl)(=nicotinic acid residue), OCO-(3-methyl-3-pyridyl), OCO-(C₁-C₆ alkyl), OCO-(C₁-C₂₁ alkenyl), OCO-NH-(C₁-C₆ alkyl), OCO-(CH₂)ₓ-NH-COO-(C₁-C₆ alkyl), O-CH₂-O-(C₁-C₆ alkyl), O-(CH₂)ₓ-OCO-(C₁-C₆ alkyl),O-(CH₂)ₓ-OCO-(CH₂)ₓ-N-COO-(C₁-C₆ alkyl), O-(CH₂)ₓ-OCO-(CH₂)_{y}-aryl, OCOO-(C₁-C₆ aminalkyl), OCOO-(CH₂)ₓ-tetrahydrofuranyl, or a sugar, preferably glucuronic acid residue, wherein x=1, 2, 3 or 4 and y=0, 1, 2, 3 or 4
R2 = H, CH₃, CO-(C₁-C₆ alkyl), CH₂-OCO-(CH₂)ₓ-aryl, or a sugar, preferably glucuronic acid residue
R3 = H, F or Br
R4 = H, C₁-C₆ alkyl, preferably CH₃, CO-(C₁-C₆ alkyl), CO-(3-pyridyl)(=nicotinic acid residue), CO-(3-methyl-3-pyridyl), CO-(CH-mercaptoalkyl)-(CH₂)ₓ-aryl, (CH₂)ₓ-OCO-(CH₂)ₓ-N-COO-(C₁-C₆ alkyl), (CH₂)ₓ-OCO-(CH-arylalkyl)-N-COO-(C₁-C₆ alkyl), wherein x=1, 2, 3 or 4
R5 = am electron pair or (CH₂)ₓ-O-(C₁-C₆ alkyl), (CH₂)ₓ-OCO-(C₁-C₆ alkyl), (CH₂)ₓ-OCO-(CH₂)ₓ-aryl, (CH₂)ₓ-OCO-(CH₂)ₓ-N-COO-(C₁-C₆ alkyl), wherein x=1, 2, 3 or 4; in case that bond <10> to <11> is a single bond the nitrogen has a positive charge and the counterion is chloride with the proviso that the compound is not Galanthamine, Norgalanthamine, Sanguinine, Norsanguinine, Lycoramine, Norlycoramine, Lycoraminone, Narwedine, Nomarwedine,3-Amino-3-deoxy-galanthamine or 3-amino-3-deoxy-1,2-dihydro-galanthamine as a pro-drug or medicament with improved blood-brain barrier permeability compared to Galanthamine.

5. Derivative of claim 4 for the preparation of a medicament for the treatment of a neurodegenerative or psychiatric or neurological disease associated with a cholinergic deficit.

6. Derivative according to claim 4 or 5, whereby the derivative is selected from the group provided in table 4.

7. Derivative of the formula (III) 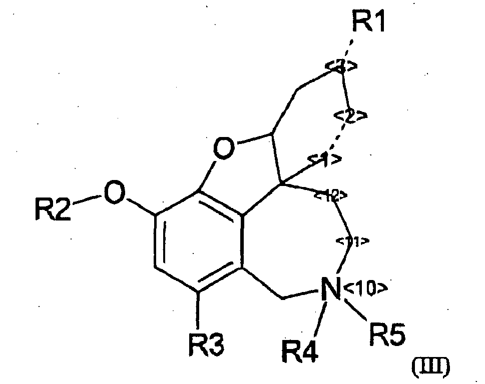 wherein the bonding <1> to <2> and <3> to R1 and <10> to <11> and residues R1, R2, R3, R4 and R5 are selected in a way that derivatives of table 4 are obtained.

8. Pharmaceutical composition comprising a derivative according to any of claims 4 to 7 or a pharmaceutically acceptable salt thereof.

9. Pharmaceutical composition according to claim 8, further comprising a pharmaceutically acceptable carrier.

10. Pharmaceutical composition according to claims 8 or 9 for the treatment of a neurodegenerative or psychiatric or neurological disease associated with a cholinergic deficit.

11. Derivative of claim 5 or pharmaceutical composition of claim 10 wherein the disease is selected from Alzheimer's and Parkinson's disease, other types of dementia, schizophrenia, epilepsy, stroke, poliomyelitis, neuritis, myopathy, oxygen and nutrient deficiencies in the brain after hypoxia, anoxia, asphyxia, cardiac arrest, chronic fatique syndrome, various types of poisoning, anesthesia, particularly neuroleptic anesthesia, spinal cord disorders, inflammation, particularly central inflammatory disorders, postoperative delirium and/or subsyndronal postoperative delirium, neuropathic pain, subsequences of the abuse of alcohol and drugs, addictive alcohol and nicotine craving, and subsequences of radiotherapy.
